(19) Europäisches Patentamt / European Patent Office / Office européen des brevets

(11) **EP 2 554 124 B1**

(12) **EUROPEAN PATENT SPECIFICATION**

(45) Date of publication and mention
of the grant of the patent:
**01.01.2014 Bulletin 2014/01**

(51) Int Cl.:
***A61B 8/00*** *(2006.01)*    ***A61B 8/08*** *(2006.01)*

(21) Application number: **11840691.7**

(22) Date of filing: **11.11.2011**

(86) International application number:
**PCT/JP2011/076602**

(87) International publication number:
**WO 2012/063975 (18.05.2012 Gazette 2012/20)**

(54) **Ultrasound observation device with the associated method and operating program**

Ultraschallbeobachtungsvorrichtung mit dem entsprechenden Verfahren und Betriebsprogramm

Dispositif d'observation échografique ainsi que les procédé et programme associés

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2010 JP 2010253285**

(43) Date of publication of application:
**06.02.2013 Bulletin 2013/06**

(73) Proprietor: **OLYMPUS MEDICAL SYSTEMS CORP. Tokyo 151-0072 (JP)**

(72) Inventor: **MIYAKI, Hironaka Tokyo 151-0072 (JP)**

(74) Representative: **Gunzelmann, Rainer Wuesthoff & Wuesthoff Patent- und Rechtsanwälte Schweigerstraße 2 81541 München (DE)**

(56) References cited:
**WO-A2-2004/093671       JP-A- 2004 049 925**
**JP-A- 2005 253 827        JP-A- 2007 097 671**
**JP-A- 2007 524 431        JP-A- 2009 523 059**
**US-A1- 2004 019 276       US-A1- 2004 059 224**
**US-A1- 2006 079 780       US-B1- 6 558 324**

## Description

Field

The present invention relates to an ultrasonic observation apparatus for observing a specimen tissue by use of ultrasonic wave, an operation method of the ultrasonic observation apparatus, and an operating program of the ultrasonic observation apparatus.

Background

There has conventionally been known an ultrasonic elastography as an examination technology of breast cancer by use of ultrasonic wave (e.g., see Patent Literature 1). The ultrasonic elastography is a technique of utilizing a phenomenon that a hardness of a cancer or a tumor tissue in a living body is different depending on a disease progression or on the living body. In this technique, a distorted amount or elasticity of a body tissue in an examination region is measured by use of ultrasonic wave with the examination region being externally pressed, and the result of the measurement is displayed as a tomographic view.

Document US 2006/0079780 A1 discloses an ultrasonic imaging apparatus capable of generating ultrasonic images including boundaries between different tissues and regions divided by the boundaries in which the property of boundaries and the respective tissues can be distinctively identified. In a distance correction process, a distance attenuation of ultrasonic echoes that varies depending on the reception depth and positional relationship with respective ultrasonic transducers are corrected.

Citation List

Patent Literature

Patent Literature 1: WO 2005/122906 A

Summary

Technical Problem

However, in the abovementioned ultrasonic elastography, there is a problem in that it is difficult to transmit applied pressure to the lower part of a vascular channel, such as a blood vessel or a lymph channel. Therefore, when a tumor is formed near the vascular channel,

a border of the tumor becomes indistinct, which makes it difficult to distinguish tumor invasion in the vascular channel. Thus, in the ultrasonic elastography, it is sometimes difficult to observe a specimen or distinct tissue characterization with accuracy.

Moreover, in the ultrasonic elastography, there is another problem in that the reliability of a measurement result is low because pressure or compression speed applied by an examiner varies from person to person when compression is applied to the screening location.

The present invention has been made in view of the above and it is an object of the present invention to provide an ultrasonic observation apparatus, an operation method of the ultrasonic observation apparatus, and an operating program of the ultrasonic observation apparatus capable of observing a specimen with accuracy and enhancing the observation result in terms of reliability.

Solution to Problem

Some or all of the above problems are overcome by an ultrasonic observation apparatus according to claim 1, an operation method of an ultrasonic observation apparatus according to claim 14, and operation program of an ultrasonic observation apparatus according to claim 15.

In order to solve the abovementioned problem and achieve the object, an ultrasonic observation apparatus according to the invention transmits an ultrasonic wave to a specimen and receives the ultrasonic wave reflected on the specimen. The ultrasonic observation apparatus includes: a frequency analyzing unit configured to calculate a frequency spectrum by analyzing a frequency of the received ultrasonic wave; a feature data extracting unit configured to extract feature data of the frequency spectrum by performing an approximation to the frequency spectrum calculated by the frequency analyzing unit; a storage unit configured to store feature data of a frequency spectrum extracted based upon the ultrasonic wave reflected respectively from plural known specimens, and to store the plural known specimens as being classified into plural groups based upon information of each of the known specimens; a degree-of-association calculating unit configured to calculate a degree of association between the feature data extracted by the feature data extracting unit and each group by using a statistics of a population including at least the feature data of each group stored in the storage unit; an image processing unit configured to generate ultrasonic image data having a display manner corresponding to the feature data of the specimen based upon the calculation result of the degree-of-association calculating unit; and a display unit configured to be capable of displaying the image corresponding to the ultrasonic image data generated by the image processing unit.

In the ultrasonic observation apparatus according to the invention, the feature data extracting unit extracts plural feature data. The storage unit stores an average of the respective feature data in each of the plural groups. The degree-of-association calculating unit sets feature data space having at least any one of the plural feature data as a component, and calculates, as the degree of association, a distance on the feature data space between a specimen point that has a feature data, which serves as the component of the feature data space, out of the feature data of the frequency spectrum of the specimen as a coordinate on the feature data space, and a known specimen average point that has an average of the feature data, which serve as the component of the feature data space, out of the feature data in the group of the plural known specimens as a coordinate on the feature data space.

In the ultrasonic observation apparatus according to the invention, the image processing unit determines a pixel value of the ultrasonic image data according to the distance between the specimen point and the known specimen average point.

In the ultrasonic observation apparatus according to the invention, the degree-of-association calculating unit calculates a standard deviation of a feature data in a new population formed by adding a feature data of the specimen to the feature data belonging to the same group, and calculates the difference between the standard deviation and the standard deviation of the feature data belonging to the same group as the degree of association.

In the ultrasonic observation apparatus according to the invention, the image processing unit determines the pixel value of the ultrasonic image data according to the difference between the standard deviation of the feature data in the new population and the standard deviation of the feature data belonging to the same group.

In the ultrasonic observation apparatus according to the invention, the plural specimens having the feature data belonging to the same group have the same tissue characterization.

In the ultrasonic observation apparatus according to the invention, the feature data extracting unit extracts the feature data by performing an attenuation correction process for reducing a contribution of an attenuation caused according to a reception depth and a frequency of the ultrasonic wave during the propagation of the ultrasonic wave to the frequency spectrum calculated by the frequency analyzing unit, and by performing an approximation process to the frequency spectrum.

In the ultrasonic observation apparatus according to the invention, the feature data extracting unit includes: an approximation unit configured to extract pre-correction feature data, which is an amount before the attenuation correction process, through an execution of the approximation process to the frequency spectrum calculated by the frequency analyzing unit; and an attenuation correction unit configured to extract the feature data of the frequency spectrum by performing the attenuation correction process to the pre-correction feature data extracted by the approximation unit.

In the ultrasonic observation apparatus according to the invention, the feature data extracting unit includes: an attenuation correction unit configured to execute the attenuation correction process to the frequency spectrum; and an approximation unit configured to extract the feature data of the frequency spectrum by performing the approximation process to the frequency spectrum that is corrected by the attenuation correction unit.

In the ultrasonic observation apparatus according to the invention, the approximation unit approximates the frequency spectrum with a polynomial according to a regression analysis.

In the ultrasonic observation apparatus according to the invention, the approximation unit approximates the frequency spectrum with a primary expression, and extracts plural feature data including at least two of a slope of the primary expression, an intercept of the primary expression, and an intensity determined by using the slope, the intercept, and a specific frequency included in a frequency band of the frequency spectrum.

In the ultrasonic observation apparatus according to the invention, the attenuation correction unit makes a great correction, as the reception depth of the ultrasonic wave is larger.

An operation method according to the invention is a method of an ultrasonic observation apparatus that transmits an ultrasonic wave to a specimen and receives the ultrasonic wave reflected on the specimen. The operation method includes: a frequency analyzing step including calculating a frequency spectrum by analyzing a frequency of the received ultrasonic wave by a frequency analyzing unit; a feature data extracting step including extracting feature data of the frequency spectrum by performing an approximation to the frequency spectrum calculated at the frequency analyzing step, by a feature data extracting unit; a degree-of-association calculating step including reading a feature data of each group from a storage unit that stores feature data of a frequency spectrum extracted based upon the ultrasonic wave reflected respectively from plural known specimens and that stores the plural known specimens as being classified into plural groups based upon information of each of the known specimens, and including calculating a degree of association between the feature data extracted by the feature data extracting unit and each group by using a statistics of a population including at least the feature data of each group, by a degree-of-association calculating unit; an image processing step including generating ultrasonic image data having a display manner corresponding to the feature data of the specimen based upon the calculation result obtained at the degree-of-association calculating step, by an image processing unit; and an image display step including displaying the image corresponding to the ultrasonic image data generated at the image processing step on a display unit.

An operating program according to the invention is a program of an ultrasonic observation apparatus that transmits an ultrasonic wave to a specimen and receives the ultrasonic wave reflected on the specimen. The operating program instructs the ultrasonic observation apparatus to perform: a frequency analyzing step including calculating a frequency spectrum by analyzing a frequency of the received ultrasonic wave by a frequency analyzing unit; a feature data extracting step including extracting feature data of the frequency spectrum by performing an approximation to the frequency spectrum calculated at the frequency analyzing step, by a feature data extracting unit; a degree-of-association calculating step including reading a feature data of each group from a storage unit that stores feature data of a frequency spectrum extracted based upon the ultrasonic wave reflected respectively from plural known specimens and that stores the plural known specimens as being classified into plural groups based upon information of each of the known specimens, and including calculating a degree of association between the feature data extracted by the feature data extracting unit and each group by using a statistics of a population including at least the feature data of each group, by a degree-of-association calculating unit; an image processing step including generating ultrasonic image data having a display manner corresponding to the feature data of the specimen based upon the calculation result obtained at the degree-of-association calculating step, by an image processing unit; and an image display step including displaying the image corresponding to the ultrasonic image data generated at the image processing step on a display unit. Advantageous Effects of Invention

According to the present invention, plural known specimens are classified into plural groups based upon the feature data of the frequency spectrum extracted on the basis of the ultrasonic wave reflected on each of the plural known specimens, and the degree of association between the feature data of the specimen extracted based upon the frequency spectrum of the ultrasonic signal and each group by using the statistic of the population including the feature data of each group. The ultrasonic image data having the display manner corresponding to the feature data of the specimen is generated based upon the calculation result. Accordingly, the difference in the tissue can clearly be distinguished without using the distorted amount or elasticity of the living tissue. Brief Description of Drawings

FIG. 1 is a block diagram illustrating a configuration of an ultrasonic observation apparatus according to a first embodiment of the present invention.

FIG. 2 is a flowchart illustrating an outline of a process of the ultrasonic observation apparatus according to the first embodiment of the present invention.

FIG. 3 is a view illustrating a display example of a B-mode image on a display unit of the ultrasonic observation apparatus according to the first embodiment of the present invention.

FIG. 4 is a flowchart illustrating an outline of a process executed by a frequency analyzing unit in the ultrasonic observation apparatus according to the first embodiment of the present invention.

FIG. 5 is a view schematically illustrating a data array of one sound ray.

FIG. 6 is a view illustrating an example (first example) of a frequency spectrum calculated by the frequency analyzing unit in the ultrasonic observation apparatus according to the first embodiment of the present invention.

FIG. 7 is a view illustrating an example (second example) of a frequency spectrum calculated by the frequency analyzing unit in the ultrasonic observation apparatus according to the first embodiment of the present invention.

FIG. 8 is a view illustrating a new straight line determined by a feature data after an attenuation correction is performed to the feature data related to the straight line illustrated in FIG. 6.

FIG. 9 is a flowchart illustrating an outline of a process executed by a degree-of-association calculating unit in the ultrasonic observation apparatus according to the first embodiment of the present invention.

FIG. 10 is a view illustrating an example of a feature data space set by the degree-of-association calculating unit in the ultrasonic observation apparatus according to the first embodiment of the present invention.

FIG. 11 is a view illustrating an example of a way of assigning a color component to each pixel in calculation result display image data.

FIG. 12 is a view illustrating another example of a way of assigning a color component to each pixel in calculation result display image data.

FIG. 13 is a view illustrating a display example of a calculation result display image displayed on the display unit in the ultrasonic observation apparatus according to the first embodiment of the present invention.

FIG. 14 is a view schematically illustrating the image illustrated in FIG. 13 in black and white.

FIG. 15 is a view describing an effect of the attenuation correction process executed by the ultrasonic observation apparatus according to the first embodiment of the present invention.

FIG. 16 is a flowchart illustrating an outline of a process of an ultrasonic observation apparatus according to a second embodiment of the present invention.

FIG. 17 is a view schematically illustrating an outline of an attenuation correction process executed by the ultrasonic observation apparatus according to the second embodiment of the present invention.

FIG. 18 is a view describing a feature data space set by a degree-of-association calculating unit in an ultrasonic observation apparatus according to a third embodiment of the present invention, and a standard deviation of a feature point in the feature data space.

FIG. 19 is a view illustrating a relationship between an absolute value of a difference in the standard deviation in the feature data space and a magnitude of a red component.

Description of Embodiments

Embodiments for carrying out the present invention (hereinafter referred to as "embodiments") will be described below with reference to the attached drawings.

(First Embodiment)

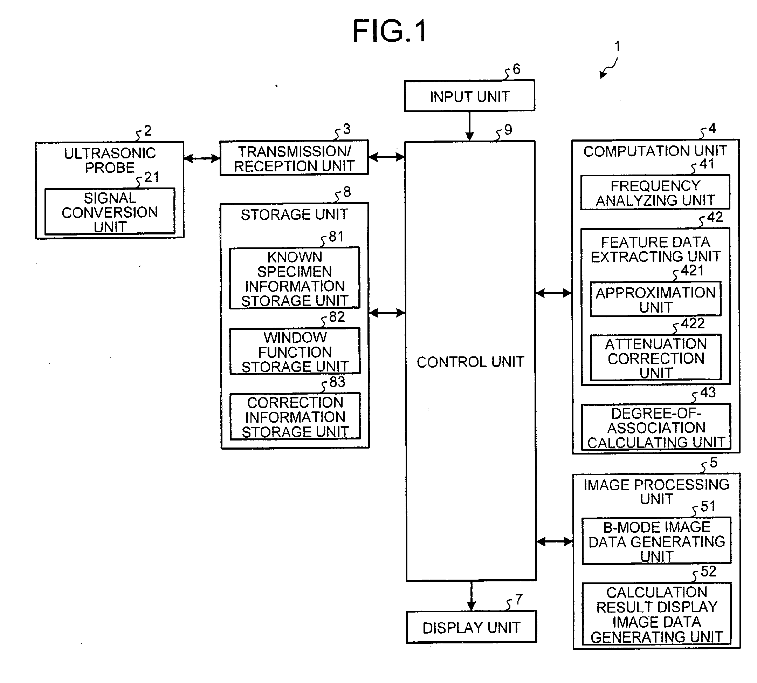

FIG. 1 is a block diagram illustrating a configuration of an ultrasonic observation apparatus according to a first embodiment of the present invention. The ultrasonic observation apparatus illustrated in FIG. 1 is an apparatus for observing a specimen by use of ultrasonic wave.

An ultrasonic observation apparatus 1 includes an ultrasonic probe 2 that outputs an ultrasonic pulse to the outside, and that receives an externally reflected ultrasonic echo, a transmission/reception unit 3 that transmits and receives an electric signal to and from the ultrasonic probe 2, a computation unit 4 that performs predetermined computation to an electric echo signal obtained by converting the ultrasonic echo, an image processing unit 5 that generates image data corresponding to the electric echo signal obtained by converting the ultrasonic echo, an input unit 6 that is realized by using an interface such as a keyboard, a mouse, or a touch panel for accepting an input of various information, a display unit 7 that is realized by using a display panel such as a liquid crystal panel or an organic EL panel, and that can display various information including the image generated by the image processing unit 5, a storage unit 8 that stores information involved with plural known specimens, and that stores the plural known specimens classified into plural groups, and a control unit 9 that controls the operation of the ultrasonic observation apparatus 1.

The ultrasonic probe 2 includes a signal conversion unit 21 that converts the electric pulse signal received by the transmission/reception unit 3 into an ultrasonic pulse (acoustic pulse signal), and that converts the ultrasonic echo reflected on an external specimen into an electric echo signal. The ultrasonic probe 2 may be the one that allows an ultrasonic vibrator to mechanically scan, or the one that allows plural ultrasonic vibrators to electronically scan.

The transmission/reception unit 3 is electrically connected to the ultrasonic probe 2, transmits the pulse signal to the ultrasonic probe 2, and receives an echo signal, which is a reception signal, from the ultrasonic probe 2. Specifically, the transmission/reception unit 3 generates a pulse signal based upon a waveform and transmission timing, set beforehand, and transmits the generated pulse signal to the ultrasonic probe 2.

The transmission/reception unit 3 is electrically connected to the ultrasonic probe 2, transmits the pulse signal to the ultrasonic probe 2, and receives an echo signal from the ultrasonic probe 2. Specifically, the transmission/reception unit 3 generates a pulse signal based upon a waveform and transmission timing, set beforehand, and transmits the generated pulse signal to the ultrasonic probe 2. The transmission/reception unit 3 performs a process such as an amplification or filtering to the received echo signal, and then, generates a digital RF signal through an A/D conversion. Then, it outputs the digital RF signal. When the ultrasonic probe 2 is configured to electronically scan plural ultrasonic vibrators, the transmission/reception unit 3 has a multichannel circuit for synthesizing beams corresponding to the plural ultrasonic vibrators.

The computation unit 4 includes a frequency analyzing unit 41 that performs fast Fourier transform (FFT) to the digital RF signal outputted from the transmission/reception unit 3 so as to analyze a frequency of the echo signal, a feature data extracting unit 42 that performs an attenuation correction process for reducing a contribution of an attenuation generated according to the reception depth and the frequency of the ultrasonic wave, and an approximation process to the frequency spectrum (power spectrum) calculated by the frequency analyzing unit 41 during the propagation of the ultrasonic wave, in order to extract a feature data of a specimen, and a degree-of-association calculating unit 43 that calculates a degree of association between the feature data extracted by the feature data extracting unit 42 and each of the plural groups stored in the storage unit 8.

The frequency analyzing unit 41 calculates the frequency spectrum of each sound ray (line data) by performing the fast Fourier transform to a FFT data group including predetermined amount of data. The frequency spectrum indicates a different tendency depending upon a tissue characterization of the specimen. This is because the frequency spectrum has a correlation with the size of the specimen serving as a scattering substance scattering the ultrasonic wave, a density, and an acoustic impedance.

The feature data extracting unit 42 includes an approximation unit 421 that calculates a pre-correction feature data, which is an amount before the execution of the attenuation correction process, by performing the approximation process to the frequency spectrum calculated by the frequency analyzing unit 41, and an attenuation correction unit 422 that extracts the feature data by performing the attenuation correction process to the pre-correction feature data approximated by the approximation unit 421.

The approximation unit 421 approximates the frequency spectrum with a primary expression according to a regression analysis, thereby extracting the pre-correction feature data characterizing the approximated primary expression. Specifically, the approximation unit 421 calculates a slope $a_0$ and an intercept $b_0$ of the primary expression according to the regression analysis, and calculates the intensity of a specific frequency within a frequency band of the frequency spectrum as the pre-correction feature data. In the first embodiment, the approximation unit 421 is supposed to calculate an intensity (Mid-band fit) $c_0 = a_0 f_{MID} + b_0$ at a center frequency $f_{MID} = (f_{LOW} + f_{HIGH})/2$. However, this is only one example. The "intensity" here means any one of parameters including a voltage, power, sound pressure, and acoustic energy.

Among the three feature data, the slope $a_0$ has a correlation with the size of the scattering body of the ultrasonic wave, and it is generally considered that the slope has a smaller value, as the size of the scattering body is large. The intercept $b_0$ has a correlation with the size of the scattering body, the difference in the acoustic impedance, and the density (concentration) of the scattering body. Specifically, it is considered that the intercept $b_0$ has a larger value, as the size of the scattering body is large, as the acoustic impedance is large, and as the density (concentration) of the scattering body is large. The intensity at the center frequency $f_{MID}$ (hereinafter merely referred to as "intensity") $c_0$ is an indirect parameter derived from the slope $a_0$ and the intercept $b_0$, and it gives a spectrum intensity at the center in the effective frequency band. Therefore, it is considered that the intensity $c_0$ has not only a correlation with the size of the scattering body, the difference in the acoustic impedance, and the density of the scattering body, but also a correlation with the brightness of the B-mode image to a certain extent. The approximate polynomial calculated by the feature data extracting unit 42 is not limited to the primary expression, but a second-order or higher-order approximate polynomial may be used.

The correction executed by the attenuation correction unit 422 will be described. An attenuation amount A of the ultrasonic wave can be expressed as:

$$A = 2\alpha z f \qquad (1)$$

Here, $\alpha$ is an attenuation rate, z is a reception depth of the ultrasonic wave, and f is a frequency. As is apparent from the equation (1), the attenuation amount A is in proportion to the frequency f. The specific value of the attenuation rate $\alpha$ for a living body is 0 to 1.0 (dB/cm/MHz), and more preferably 0.3 to 0.7 (dB/cm/MHz). The attenuation rate is determined according to a type of an organ to be observed. For example, when the organ to be observed is pancreas, it is determined that $\alpha = 0.6$ (dB/cm/MHz). It can be configured in the first embodiment that the value of the attenuation rate $\alpha$ can be changed by the input from the input unit 6.

The attenuation correction unit 422 corrects the pre-correction feature data (slope $a_0$, intercept $b_0$, intensity $c_0$) extracted by the approximation unit 421 as described below.

$$a = a_0 + 2\alpha z \qquad (2)$$

$$b = b_0 \qquad (3)$$

$$c = c_0 + 2\alpha z f_{MID} \quad (= a f_{MID} + b) \qquad (4)$$

As is apparent from the equations (2) and (4), the attenuation correction unit 422 makes a correction with a large correction amount, as the reception depth z of the ultrasonic wave is larger. According to the equation (3), the correction involved with the intercept is an identical transformation. This is because the intercept is not subject to the attenuation, since the intercept is the frequency component corresponding to the frequency 0 (Hz).

The degree-of-association calculating unit 43 calculates the degree of association between the feature data extracted by the feature data extracting unit 42 and each of the groups by using a statistic of a population including at least the feature data of the respective groups stored in the storage unit 8. More specifically, the degree-of-association calculating unit 43 sets a feature data space, which has at least any one of the plural feature data extracted by the feature data extracting unit 42 as a component, and calculated, as the degree of association, the distance on the feature data space between a specimen point having the feature data, serving as the component in the set feature data space, out of the feature data of the frequency spectrum of the specimen as a coordinate in the feature data space, and a known specimen average point having an average of the feature data serving as the component in the feature data space out of the feature data of the plural groups of the known specimens as the coordinate of the feature data space.

The average and standard deviation of the feature data of the frequency spectrum of the ultrasonic reception signal reflects a change in a cell level such as an enlarged nucleus, and heteromorphy of nucleus in a specimen, or a change in a tissue such as increased fibra in interstitium, or replacement of a parenchymal tissue into a fiber, and it indicates a unique value according to the tissue characterization. The "tissue characterization" here means any one of a cancer, endocrine tumor, mucinous tumor, normal tissue, and vessel channel. When the specimen is a pancreas, the tissue characterization includes chronic pancreatitis, and autoimmune pancreatitis. In view of the above circumstance, the groups stored in the storage unit 8 are desirably determined according to the type of the tissue characterization. Therefore, it is supposed in the description below that the groups stored in the storage unit 8 are determined according to the type of the tissue characterization.

The image processing unit 5 includes a B-mode image data generating unit 51 that generates B-mode image data from the echo signal, and a calculation result display image data generating unit 52 that generates calculation result display image data that is ultrasonic image data for displaying the calculation result of the degree of association by using the calculation result of the degree of association calculated by the degree-of-association calculating unit 43 and the B-mode image data generated by the B-mode image data generating unit 51.

The B-mode image data generating unit 51 performs, to the digital signal, a signal processing using a known technique such as a bandpass filter, logarithmic conversion, gain process or contrast process, and generates the B-mode image data by decimating the data according to a data step width determined by a display range of the image on the display unit 7.

The calculation result display image data generating unit 52 generates the calculation result display image data by using the B-mode image data generated by the B-mode image data generating unit 51, the feature data extracted by the feature data extracting unit 42, and the result calculated by the degree-of-association calculating unit 43.

The storage unit 8 includes a known specimen information storage unit 81 that stores information of the known specimen, a window function storage unit 82 that stores a window function used for the frequency analyzing process executed by the frequency analyzing unit 41, and a correction information storage unit 83 that stores correction information referred to during the execution of the process by the attenuation correction unit 422.

The known specimen information storage unit 81 stores the feature data of the frequency spectrum extracted from the known specimen in association with the tissue characterization of the known specimen. The known specimen information storage unit 81 also stores the average and standard deviation of the feature data of the frequency spectrum involved with the known specimen together with all data of the feature data of the known specimen, wherein the average and the standard deviation is calculated for each of the groups classified based upon the information including the tissue characterization of the known specimen. The feature data of the known specimen is extracted in the same manner as in the first embodiment. It is to be noted that the process of extracting the feature data of the known specimen is not necessarily executed by the ultrasonic observation apparatus 1. It is desirable that the information of the known specimen stored in the known specimen information storage unit 81 has high reliability for the tissue characterization. The window function storage unit 82 stores at least any one of window functions of Hamming, Hanning, and Blackman. The correction information storage unit 83 stores information involved with the transformation of the equations (2) to (4).

The storage unit 8 is realized by ROM that preliminarily stores an operating program of the ultrasonic observation apparatus according to the first embodiment or a program of starting a predetermined OS, and RAM that stores operation parameters or data for each process.

The components, other than the ultrasonic probe 2, of the ultrasonic observation apparatus 1 thus configured are realized by using a computer provided with a CPU having a computation and control functions. The CPU provided to the ultrasonic observation apparatus 1 reads the information memorized and stored in the storage unit 8 and various programs including the operating program of the ultrasonic observation apparatus from the storage unit 8, thereby executing the computation process involved with the operation method of the ultrasonic observation apparatus according to the first embodiment.

The operating program of the ultrasonic observation apparatus according to the first embodiment can be recorded on a computer-readable recording medium, such as hard disk, flash memory, CD-ROM, DVD-ROM, or flexible disk.

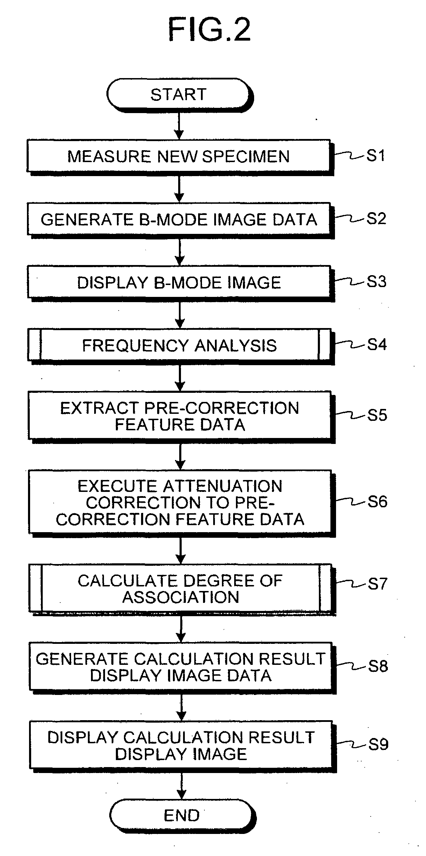

FIG. 2 is a flowchart illustrating an outline of a process of the ultrasonic observation apparatus 1 having the above-mentioned configuration. In FIG. 2, the ultrasonic observation apparatus 1 firstly measures a new specimen by the ultrasonic probe 2 (step S1).

Then, the B-mode image data generating unit 51 generates the B-mode image data by using the echo signal for the B-mode image outputted from the transmission/reception unit 3 (step S2).

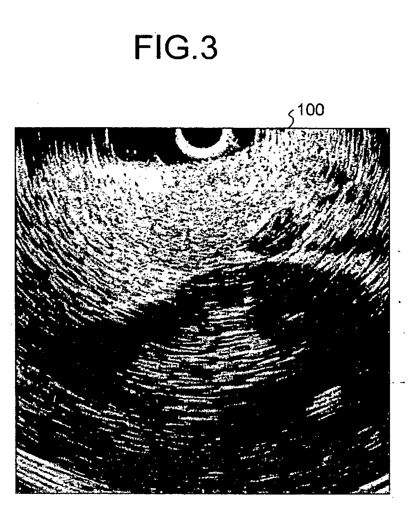

Next, the control unit 9 makes control to display the B-mode image corresponding to the B-mode image data generated by the B-mode image data generating unit 51 onto the display unit 7 (step S3). FIG. 3 is a view illustrating a display example of the B-mode image on the display unit 7. The B-mode image 100 in FIG. 3 is a grayscale image in which values of R (red), G (green), and B (blue), which are variables when an RGB color system is employed as a color space, are matched to one another.

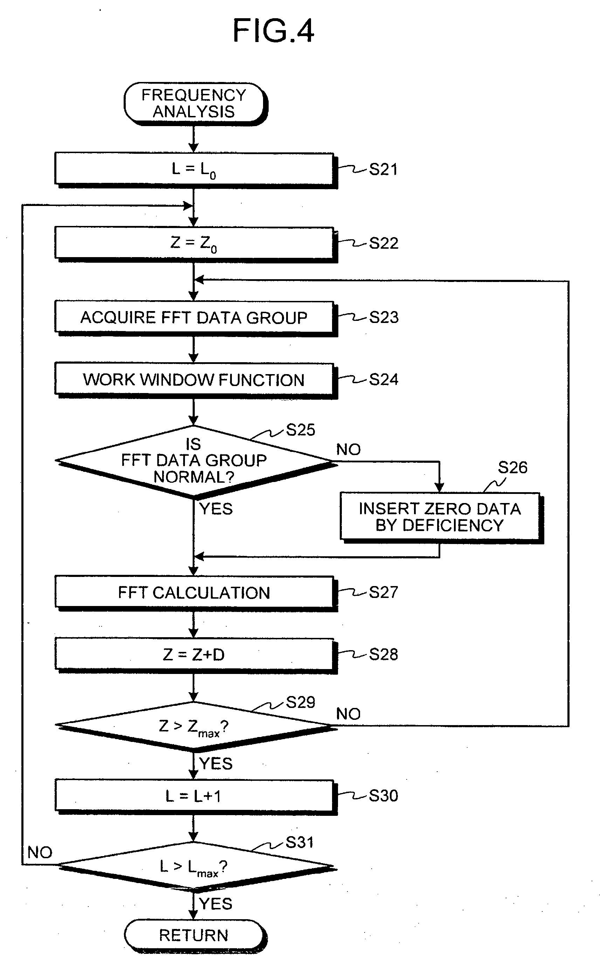

Then, the frequency analyzing unit 41 calculates the frequency spectrum by performing the frequency analysis according to the FFT calculation (step S4). The process (step S4) executed by the frequency analyzing unit 41 will be described in detail with reference to FIG. 4. Firstly, the frequency analyzing unit 41 sets a sound ray number L of a sound ray that is a subject to be analyzed to an initial value $L_0$ (step S21). The initial value $L_0$ may be applied to a sound ray that the transmission/reception unit 3 receives first, or may be applied to a sound ray corresponding to one of boundary positions on the left and on the right of a region of interest set by the input unit 6.

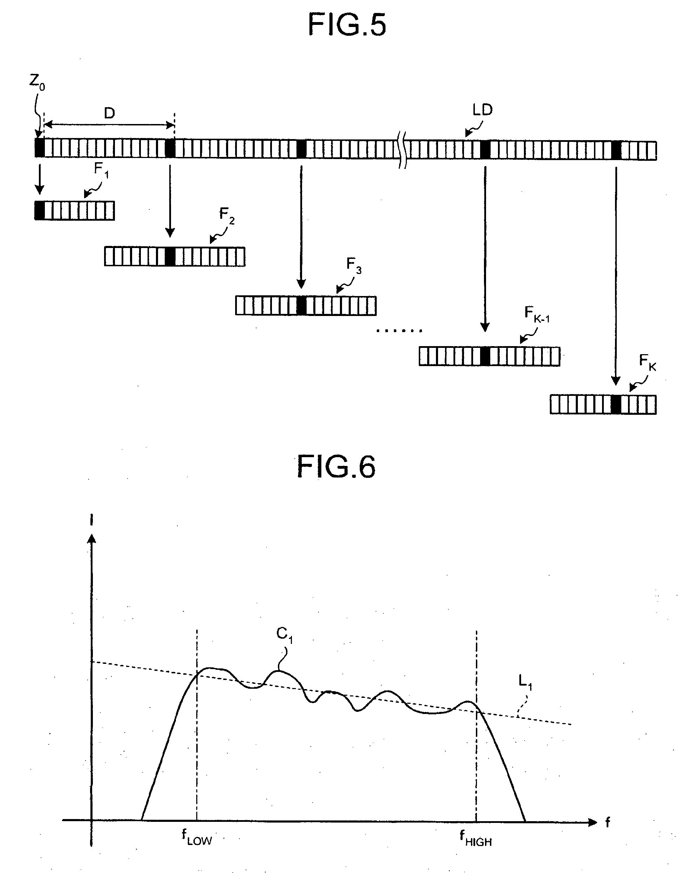

Next, the frequency analyzing unit 41 calculates all frequency spectrums on plural data positions set on one sound ray. Firstly, the frequency analyzing unit 41 sets an initial value $Z_0$ on a data position Z (corresponding to the reception depth) representative of a series of data group (FFT data group) acquired for the FFT calculation (step S22). FIG. 5 is a view schematically illustrating a data array of one sound ray. On the sound ray LD illustrated in FIG. 5, a white rectangle or a black rectangle indicates one data. The sound ray LD is discretized with a time interval corresponding to the sampling frequency (e.g., 50 MHz) in the A/D conversion executed by the transmission/reception unit 3. In FIG. 5, the first data on the sound ray LD is set as the initial value $Z_0$ on the data position Z. FIG. 5 illustrates only one example, and any position can be set as the position of the initial value $Z_0$. For example, the data position Z corresponding to the upper end of the region of interest may be set as the initial value $Z_0$.

Then, the frequency analyzing unit 41 acquires the FFT data group on the data position Z (step S23), and allows the window function stored in the window function storage unit 82 to work on the acquired FFT data group (step S24). Allowing the window function to work on the FFT data group as described above avoids that the FFT data group becomes discontinuity on the boundary, and can prevent the occurrence of artifacts.

Next, the frequency analyzing unit 41 determines whether the FFT data group on the data position Z is a normal data group or not (step S25). The FFT data group has to have a power-of-2 data number. The data number of the FFT data group is supposed to be $2^n$ (n is a positive integer) hereinafter. The case where the FFT data group is normal means that the data position Z is on the $2^{n-1}$-th position from the head of the FFT data group. In other words, the case where the FFT data group is normal means that there are $2^{n-1} - 1$ (= N) data before the data position Z, and there are $2^{n-1}$ (= M) data after the data position Z. In FIG. 5, the FFT data groups $F_2$, $F_3$, and $F_{K-1}$ are normal, but the FFT data groups $F_1$ and $F_K$ are abnormal. In FIG. 5, it is set such that n = 4 (N = 7, M = 8).

When the FFT data group on the data position Z is normal as a result of the determination in step S25 (step S25: Yes), the frequency analyzing unit 41 moves to a later-described step S27.

When the FFT data group on the data position Z is abnormal as a result of the determination in step S25 (step S25: No), the frequency analyzing unit 41 inserts zero data by a deficiency to generate the normal FFT data group (step S26). The window function is worked on the FFT data group that is determined to be abnormal in step S25 before the zero data is added. Therefore, even if the zero data is inserted to the FFT data group, a data discontinuity does not occur. The frequency analyzing unit 41 moves to the later-described step S27 after step S26.

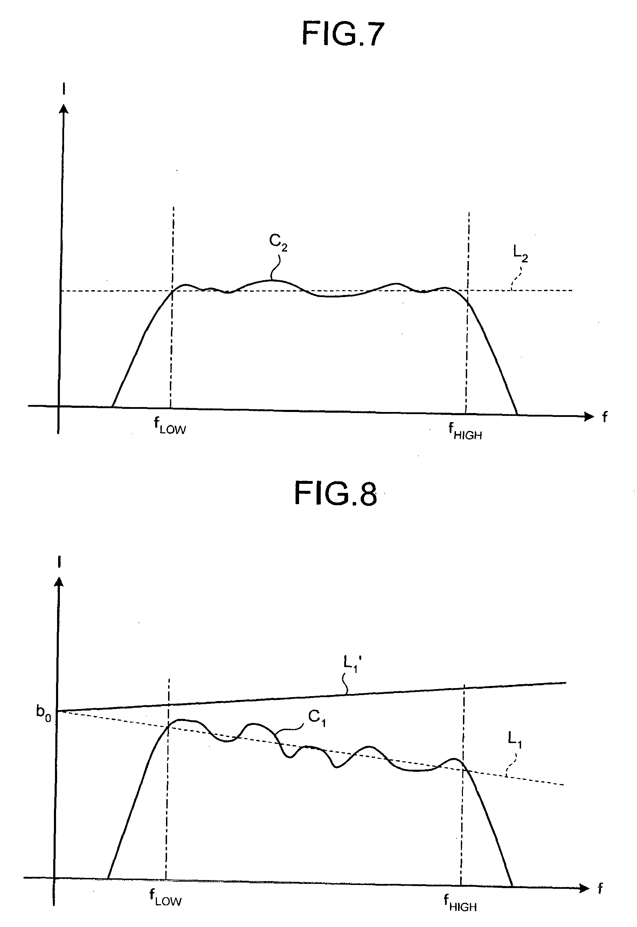

In step S27, the frequency analyzing unit 41 acquires the frequency spectrum by performing the FFT calculation by use of the FFT data group (step S27). FIGS. 6 and 7 are views illustrating the frequency spectrum calculated by the frequency analyzing unit 41. In FIGS. 6 and 7, an abscissa axis f indicates a frequency, and an ordinate axis I indicates an intensity. In frequency spectrum curves $C_1$ and $C_2$ illustrated in FIGS. 6 and 7 respectively, the lower-limit frequency $f_{LOW}$ and the upper-limit frequency $f_{HIGH}$ of the frequency spectrum are parameters determined based upon the frequency band of the ultrasonic probe 2 and the frequency band of the pulse signal transmitted by the transmission/reception unit 3. For example, they are set such that $f_{LOW}$ = 3 MHz, and $f_{HICH}$ = 10 MHz. A straight line $L_1$ illustrated in FIG. 6 and a straight line $L_2$ illustrated in FIG. 7 will be described for the later-described feature data extracting process. In the first embodiment, the curve and the straight line are composed of a set of discrete points. This is applied to the later-described embodiments.

Next, the frequency analyzing unit 41 adds a predetermined data step width D to the data position Z to calculate the data position Z of the FFT data group that is the next subject to be analyzed (step S28). The data step width D desirably agrees with the data step width utilized for generating the B-mode image data by the B-mode image data generating unit 51. However, if the computation amount of the frequency analyzing unit 41 is intended to be reduced, the data step width larger than the data step width utilized by the B-mode image data generating unit 51 may be set. FIG. 5 illustrates the case where D = 15.

Then, the frequency analyzing unit 41 determines whether the data position Z is larger than a final data position $Z_{max}$ or not (step S29). The final data position $Z_{max}$ may be a data length of the sound ray LD, or may be the data position corresponding to the lower end of the region of interest. When the data position Z is larger than the final data position $Z_{max}$ as a result of the determination (step S29: Yes), the frequency analyzing unit 41 increments the sound ray number L by 1 (step S30). On the other hand, when the data position Z is not more than the final data position $Z_{max}$ (step S29: No), the frequency analyzing unit 41 returns to step S23. In this way, the frequency analyzing unit 41 performs the FFT calculation to the $[\{(Z_{max} - Z_0)/D\} + 1]$ (= K) FFT data groups with respect to one sound ray LD. Here, [X] indicates the maximum integer not exceeding X.

When the sound ray number L to which 1 is added in step S30 is larger than a final sound ray number $L_{max}$ (step S31: Yes), the frequency analyzing unit 41 returns to the main routine illustrated in FIG. 2. On the other hand, when the sound ray number L to which 1 is added in step S30 is not more than the final sound ray number $L_{max}$ (step S31: No), the frequency analyzing unit 41 returns to step S22.

In this way, the frequency analyzing unit 41 executes the FFT calculation K times to each of $(L_{max} - L_0 + 1)$ sound rays. The final sound ray number $L_{max}$ may be applied to the final sound ray received by the transmission/reception unit 3, or may be applied to the sound ray corresponding to one of left and right boundaries of the region of interest, for example. The total number $(L_{max} - L_0 + 1) \times K$ of the FFT calculation executed to all sound rays by the frequency analyzing unit 41 is set as P below.

Subsequent to the frequency analyzing process in step S4 described above, the approximation unit 421 extracts the pre-correction feature data by performing the regression analysis to P frequency spectrums calculated by the frequency analyzing unit 41 as the approximation process (step S5). Specifically, the approximation unit 421 calculates the primary expression, which approximates the frequency spectrum in the frequency band ($f_{LOW} < f < f_{HIGH}$), according to the regression analysis, thereby extracting the slope $a_0$, the intercept $b_0$, and the intensity $c_0$ characterizing the primary expression as the pre-correction feature data. The straight line $L_1$ in FIG. 6 and the straight line $L_2$ in FIG. 7 are regression lines obtained by performing the regression analysis to the frequency spectrum curves $C_1$ and $C_2$ respectively in step S5.

Thereafter, the attenuation correction unit 422 performs the attenuation correction process to the pre-correction feature data extracted by the approximation unit 421 (step S6). For example, when the sampling frequency of the data is 50 MHz, the time interval of the data sampling is 20 (nsec). If the sound speed is supposed to be 1530 (m/sec), the sampling distance interval of the data becomes 1530 (m/sec) $\times$ 20 (nsec)/2 = 0.0153 k (mm). If the data step number from the first data of the sound ray LD to the data position of the FFT data group that is the subject for the process is k, the data position Z becomes 0.0153 k (mm). The attenuation correction unit 422 calculates the slope a, the intercept b, and the intensity c, which are the feature data of the frequency spectrum, by substituting the data position Z obtained in this way into the reception depth z in the above-mentioned equations (2) to (4). FIG. 8 is a view illustrating a straight line determined by the feature data obtained by performing the attenuation process to the feature data involved with the straight line $L_1$ illustrated in FIG. 6. The equation representing the straight line $L_1'$ illustrated in FIG. 8 is:

$$I = af + b = (a_0 + 2\alpha Z)f + b_0 \qquad (5)$$

As is apparent from the equation (5), the straight line $L_1'$ has a slope larger than that of the straight line $L_1$, and an intercept equal to the intercept of the straight line $L_1$.

Thereafter, the degree-of-association calculating unit 43 calculates the degree of association between the specimen and the groups of the known specimens stored in the storage unit 8 based upon the feature data extracted by the feature data extracting unit 42 and the known specimen information stored in the known specimen information storage unit 81 (step S7).

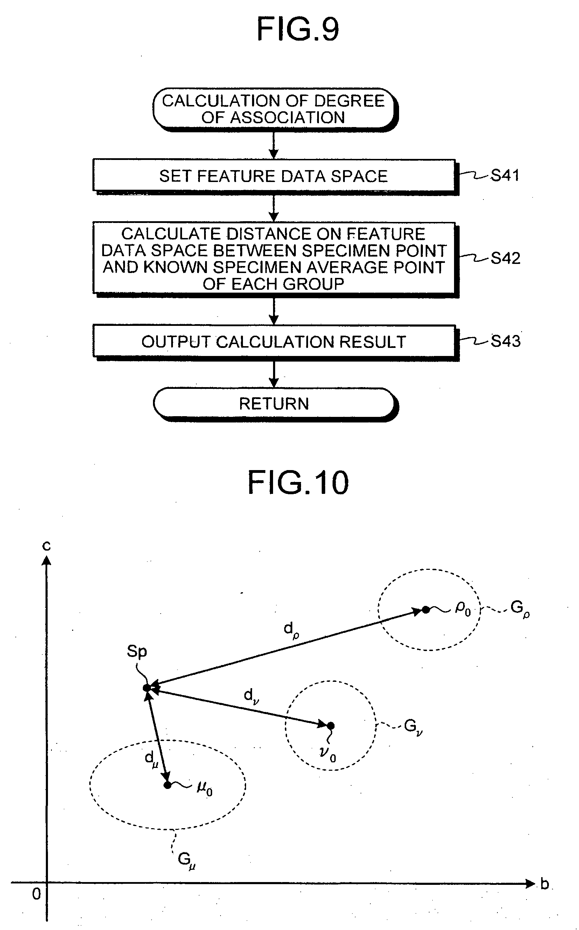

The process (step S7) executed by the degree-of-association calculating unit 43 will be described in detail with reference to the flowchart in FIG. 9. Firstly, the degree-of-association calculating unit 43 sets the feature data space used for calculating the degree of association (step S41). In the first embodiment, the independent parameters are two out of the three feature data that are the slope a, the intercept b, and the intensity c. Therefore, a two-dimensional space having any two of the three feature data as components can be set as the feature data space. Alternatively, a one-dimensional space having any one of the three feature data as a component can be set as the feature data space. In step S41, it is supposed that the feature data space to be set is determined beforehand. However, an operator may select a desired feature data space by the input unit 6.

FIG. 10 is a view illustrating an example of the feature data space set by the degree-of-association calculating unit 43. In the feature data space illustrated in FIG. 10, the abscissa axis indicates the intercept b, and the ordinate axis indicates the intensity c. A point Sp in FIG. 10 indicates a point (hereinafter referred to as a "specimen point") having the intercept b and the intensity c calculated for the specimen to be examined as a coordinate in the feature data space. Regions $G_\mu$, $G_\nu$, and $G_\rho$ illustrated in FIG. 10 respectively indicate groups in which a tissue characterization of the known specimen stored in the known specimen information storage unit 81 is $\mu$, $\nu$, and $\rho$. In the case of FIG. 10, three groups $G_\mu$, $G_\nu$, and $G_\rho$ are present in the regions not intersecting with one another in the feature data space.

In the first embodiment, even in obtaining the feature data of the known specimen, the grouping is executed by using the feature data, which is obtained by performing the attenuation correction to the pre-correction feature data of the frequency spectrum obtained by the frequency analysis, as an index. Therefore, the different groups can clearly be separated. Since the feature data to which the attenuation correction is performed is used in the first embodiment, in particular, the regions of the respective groups can be obtained as being clearly separated, compared to the case where the feature data that is extracted without being subjected to the attenuation correction is used.

After step S41, the degree-of-association calculating unit 43 calculates distances $d_\mu$, $d_\nu$, and $d_\rho$ on the feature data space between the specimen point Sp and respective points $\mu_0$, $\nu_0$, and $\rho_0$ (these points are hereinafter referred to as "known specimen average points") that are included in the groups $G_\mu$, $G_\nu$, and $G_\rho$, and that have an average of the intercepts b and the intensities c of the frequency spectrum of the FFT data group as the coordinate in the feature data space (step S42). The degree-of-association calculating unit 43 calculates the distance between all specimen points of the frequency spectrum and the known specimen average point on the feature data space in step S42. If the scale of the b-axis component and the scale of the c-axis component on the feature data space are greatly different, a weighting is desirably executed in order to equalize the contribution of each distance, as needed.

Next, the degree-of-association calculating unit 43 outputs the distance calculated in step S42 as the calculation result (step S43). Thus, the degree-of-association calculation process in step S7 is ended.

The degree-of-association calculating unit 43 may determine the tissue characterization of the specimen based upon the calculation result subsequent to step S43. In this case, however, the known specimen information storage unit 81 has to store the feature data of the known specimen and the tissue characterization in association with each other. When the specimen point Sp is extremely apart from the known specimen average points $\mu_0$, $\nu_0$, and po, the reliability of the determination result of the tissue characterization is low even if the minimum values of the distances $d_\mu$, $d_\nu$, and $d_\rho$ are obtained. Therefore, when the distances $d_\mu$, $d_\nu$, and $d_\rho$ are greater than a predetermined threshold value, the degree-of-association calculating unit 43 may output an error signal. When any two of the distances $d_\mu$, $d_\nu$, and $d_\rho$ assume the minimum value, the degree-of-association calculating unit 43 may select all tissue characterizations corresponding to the minimum value as candidates, or may select any one of the tissue characterization according to a prescribed rule. In the latter case, a method in which a priority order of the tissue characterization having high malignancy such as a cancer is set high may be employed. When any two or more of the distances $d_\mu$, $d_\nu$, and $d_\rho$ assume the minimum value, the degree-of-association calculating unit 43 may output an error signal.

After step S7 described above, the calculation result display image data generating unit 52 generates the calculation result display image data by using the B-mode image data generated by the B-mode image data generating unit 51, the feature data calculated by the feature data extracting unit 42, and the result calculated by the degree-of-association calculating unit 43 (step S8). In this case, the calculation result display image data generating unit 52 assigns a color component (a variable composing a color space) serving as visual information to each pixel according to the distance between the specimen point Sp and the known specimen average point on the feature data space. For example, in the feature data space illustrated in FIG. 10, a red component (R) is assigned to the distance $d_\mu$ between the specimen point Sp and the known specimen average point $\mu_0$, a green component (G) is assigned to the distance $d_\nu$ between the specimen point Sp and the known specimen average point $\nu_0$, and a blue component (B) is assigned to the distance $d_\rho$ between the specimen point Sp and the known specimen average point $\rho_0$, and the pixel value is changed according to the distance.

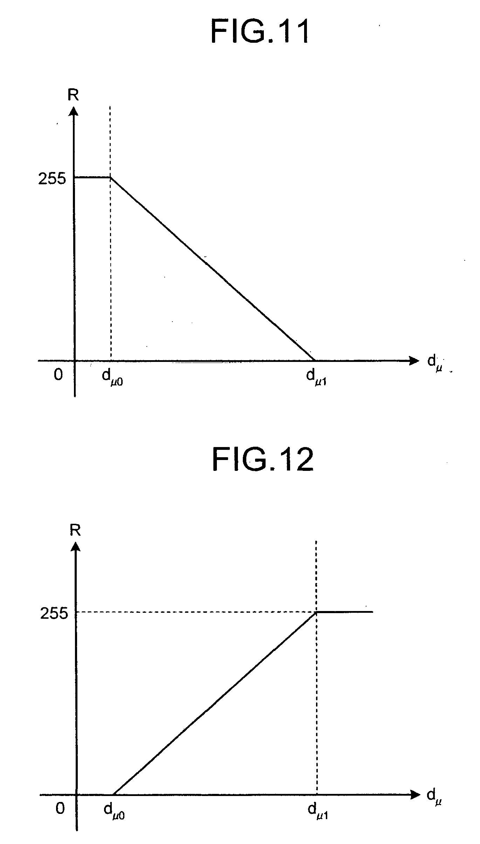

FIG. 11 is a view illustrating an example of a way of assigning the color component to each pixel in the calculation result display image data. Specifically, FIG. 11 is a view illustrating the relationship between the distance $d_\mu$ between the specimen point Sp and the known specimen average point $\mu_0$ on the feature data space and the magnitude of the red component R. In FIG. 11, the red component R assumes 255 (the maximum value in 8-bit notation), when the distance $d_\mu$ satisfies $0 \leq d_\mu \leq d_{\mu 0}$. The value of the red component R linearly decreases when the distance $d_\mu$ satisfies $d_{\mu 0} < d_\mu \leq d_{\mu 1}$, and when the distance $d_\mu$ satisfies $d_\mu > d_{\mu 1}$, it becomes zero. The magnitude of the red component has been described above. The magnitudes of the green component and the blue component can be determined according to the distances $d_\nu$ and $d_\rho$, respectively. The color space may be composed of variables of complementary colors such as cyan, magenta, and yellow, instead of the variables of the RGB color system. Alternatively, three attributes of light (color phase, brightness, saturation), which are different from the others, may be assigned to the distances $d_\mu$, $d_\nu$, and $d_\rho$. In this case, the attribute value is changed according to the distance. A pattern determined according to the distance may be assigned to any one of the distances $d_\mu$, $d_\nu$, and $d_\rho$.

FIG. 12 is a view illustrating another example of a way of assigning the color component to each pixel in the calculation result display image data, and it is a view illustrating the relationship between the distance $d_\mu$ and the magnitude of the red component R as in FIG. 11. In FIG. 12, the value of the red component R is zero when the distance

$d_\mu$ satisfies $0 \leq d_\mu \leq d_{\mu 0}$. When the distance $d_\mu$ satisfies $d_{\mu 0} < d_\mu \leq d_{\mu 1}$, the red component R linearly increase, and when the distance $d_\mu$ satisfies $d_\mu > d_{\mu 1}$, it assumes 255. As described above, the magnitude of the color component assigned to each pixel in the calculation result display image data may arbitrarily be set.

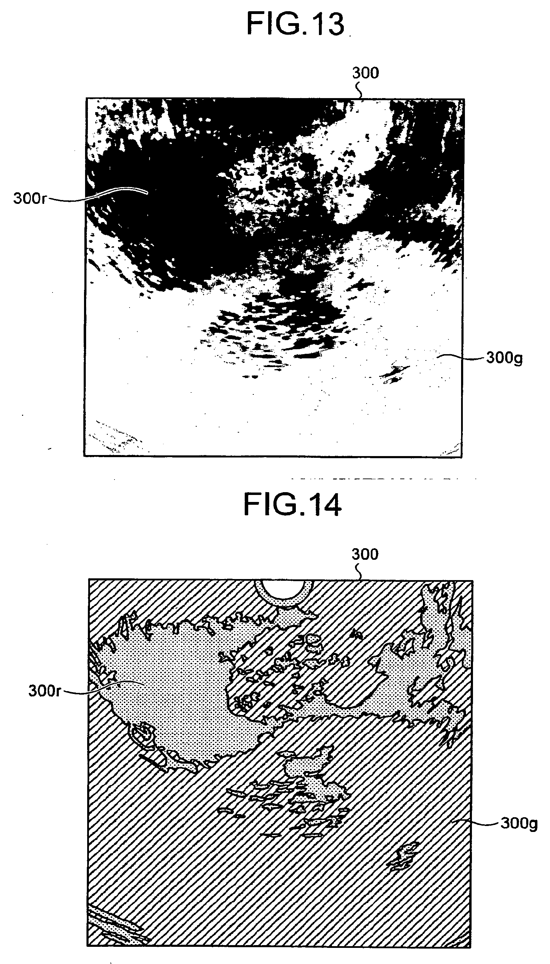

The display unit 7 displays the calculation result display image generated by the calculation result display image data generating unit 52 (step S9). FIG. 13 is a view illustrating a display example of the calculation result display image displayed on the display unit 7. FIG. 14 is a view schematically illustrating the image illustrated in FIG. 13 in black and white. Compared to the B-mode image 100, an calculation result display image 300 illustrated in these figures are colored, so that the color difference according to each group is made clear. More specifically, the calculation result display image data 300 roughly includes a green region 300g and a red region 300r. The boundary between two regions is illustrated with a yellow color (not illustrated in FIG. 14). As illustrated in FIG. 14, each region is not formed of a single color. For example, the green region 300g is a region where greenish pixels are collected. Similarly, the red region 300r is a region where reddish pixels are collected. Therefore, an observer can clearly recognize the difference in the groups, i.e., the difference in the tissue characterization.

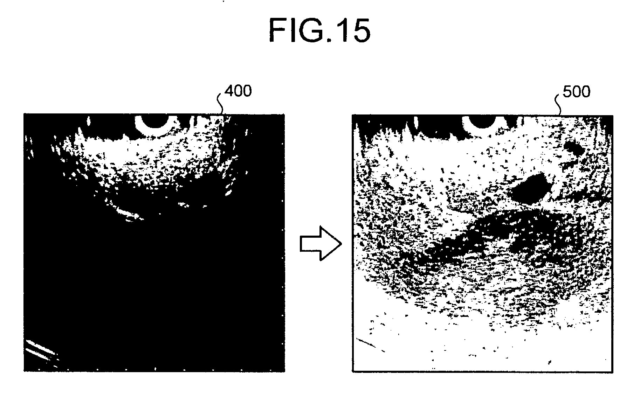

FIG. 15 is a view describing the effect of the attenuation correction process executed by the ultrasonic observation apparatus 1. An image 400 in FIG. 15 is a calculation result display image to which the attenuation correction is not executed. The calculation result display image in this case is a grayscale image in which the intercept b is equally assigned to R (red), G (green), and B (blue) with respect to the B-mode image generated by the B-mode image data generating unit 51. In the calculation result display image 400, the signal intensity is reduced due to the influence of the attenuation on the region (lower region in FIG. 14) having the large reception depth, so that the image becomes dark. On the other hand, a calculation result display image 500 obtained by performing the attenuation correction to the same B-mode image has uniform brightness over the screen.

According to the first embodiment of the present invention, plural known specimens are classified into plural groups based upon the feature data of the frequency spectrum extracted on the basis of the ultrasonic wave reflected on each of the plural known specimens, and the degree of association between the feature data of the specimen extracted based upon the frequency spectrum of the ultrasonic signal and each group by using the statistic of the population including the feature data of each group. The ultrasonic image data having the display manner corresponding to the feature data of the specimen is generated based upon the calculation result. Accordingly, the difference in the tissue can clearly be distinguished without using the distorted amount or elasticity of the living tissue. Consequently, the specimen can precisely be observed, whereby the reliability in the observation result can be enhanced.

In the first embodiment, the attenuation correction is performed to the pre-correction feature data extracted from the frequency spectrum. Therefore, the influence of the attenuation caused by the propagation of the ultrasonic wave can be eliminated, whereby the tissue characterization can be distinguished with higher accuracy.

(Second Embodiment)

In the second embodiment of the present invention, the feature data extracting process executed by the feature data extracting unit is different from that in the first embodiment. The configuration of the ultrasonic observation apparatus according to the second embodiment is the same as that of the ultrasonic observation apparatus 1 in the first embodiment. Therefore, the components corresponding to those in the ultrasonic observation apparatus 1 are identified by the same numerals in the description below.

In the feature data extracting process in the second embodiment, the attenuation correction unit 422 firstly performs the attenuation correction process to the frequency spectrum calculated by the frequency analyzing unit 41. Thereafter, the approximation unit 421 performs the approximation process to the frequency spectrum to which the attenuation correction is performed by the attenuation correction unit 422, thereby extracting the feature data of the frequency spectrum.

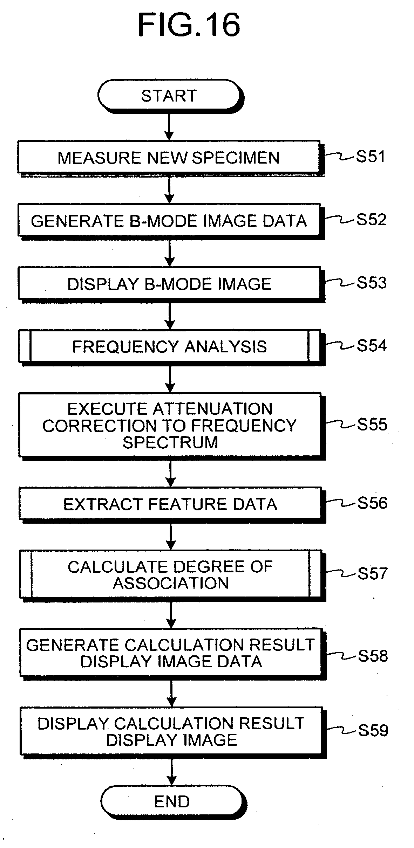

FIG. 16 is a flowchart illustrating the outline of the process of the ultrasonic observation apparatus according to the second embodiment. In FIG. 16, the processes in steps S51 to S54 respectively correspond to the processes in steps S1 to S4 in FIG. 2.

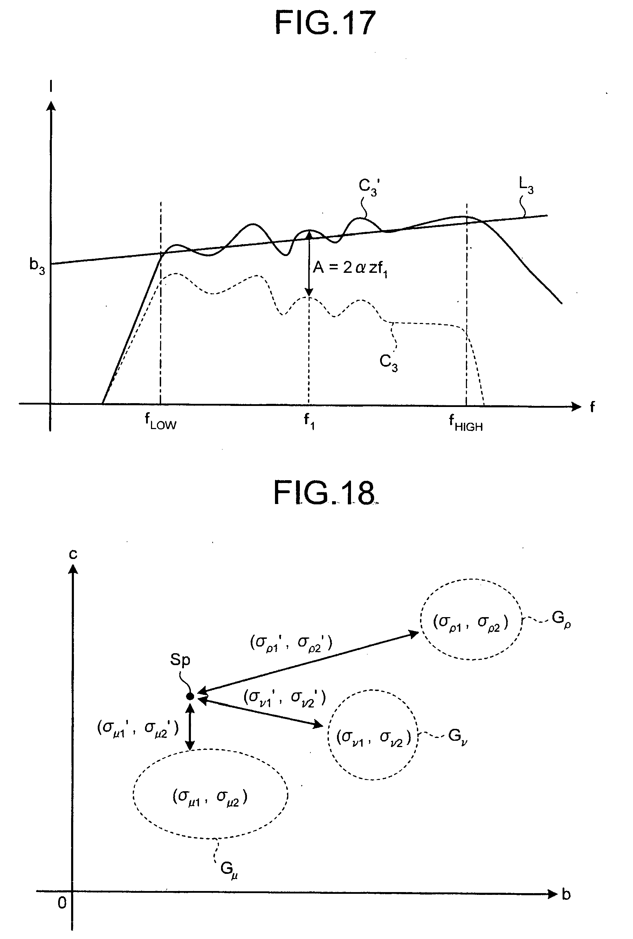

In step S55, the attenuation correction unit 422 performs the attenuation correction process to the frequency spectrum calculated by the frequency analyzing unit 41 according to the FFT calculation (step S55). FIG. 17 is a view schematically illustrating the outline of the process in step S55. As illustrated in FIG. 17, the attenuation correction unit 422 performs the correction in which the attenuation amount A in the above-mentioned equation (1) is added to the intensity I to all frequencies f, thereby obtaining a new frequency spectrum curve $C_3'$ with respect to the frequency spectrum curve $C_3$. Thus, the frequency spectrum from which the contribution of the attenuation caused by the propagation of the ultrasonic wave is reduced can be obtained.

Thereafter, the approximation unit 421 extracts the feature data of the frequency spectrum by performing the regression analysis to all frequency spectrums to which the attenuation correction is executed by the attenuation correction unit 422 (step S56). Specifically, the approximation unit 421 calculates the slope a, the intercept b, and the intensity c at the center frequency $f_{MID}$ according to the regression analysis. A straight line $L_3$ illustrated in FIG. 17 is a regression line (intercept $b_3$) obtained by performing the feature data extracting process to the frequency spectrum curve $C_3$ in step S56.

The processes in steps S57 to S59 respectively correspond to the processes in steps S7 to S9 in FIG. 2.

Even in the second embodiment, the degree-of-association calculating unit 43 can be configured to determine the tissue characterization of the specimen by using the calculated degree of association, as in the first embodiment. In this case, the known specimen information storage unit 81 stores the feature data of the known specimen in association with the tissue characterization.

According to the second embodiment of the present invention, plural known specimens are classified into plural groups based upon the feature data of the frequency spectrum extracted on the basis of the ultrasonic wave reflected on each of the plural known specimens, and the degree of association between the feature data of the specimen extracted based upon the frequency spectrum of the ultrasonic signal and each group by using the statistic of the population including the feature data of each group. The ultrasonic image data having the display manner corresponding to the feature data of the specimen is generated based upon the calculation result. Accordingly, the difference in the tissue can clearly be distinguished without using the distorted amount or elasticity of the living tissue. Consequently, the specimen can precisely be observed, whereby the reliability in the observation result can be enhanced.

In the present second embodiment, the feature data is extracted after the attenuation correction is performed to the frequency spectrum. Therefore, the influence of the attenuation caused by the propagation of the ultrasonic wave can be eliminated, whereby the tissue characterization can be distinguished with higher accuracy.

(Third Embodiment)

In the third embodiment of the present invention, the tissue characterization determining process executed by the degree-of-association calculating unit is different from that in the first embodiment. The configuration of the ultrasonic observation apparatus according to the third embodiment is the same as that of the ultrasonic observation apparatus 1 in the first embodiment. Therefore, the components corresponding to those in the ultrasonic observation apparatus 1 are identified by the same numerals in the description below.

The degree-of-association calculating unit 43 forms new populations by adding feature data (a, b, c) to the groups $G_{\mu}$, $G_{\mu}$, and $G_{\rho}$ (see FIG. 10) composing the tissue characterizations $\mu$, $\nu$, and $\rho$, and then, obtains the standard deviation for each feature data in the data composing each tissue characterization. As in the first embodiment, the degree-of-association calculating unit 43 may determine the tissue characterization of the specimen by using the calculated degree of association. In this case, the known specimen information storage unit 81 stores the feature data of the known specimen in association with the tissue characterization.

Then, the degree-of-association calculating unit 43 calculates the difference (hereinafter merely referred to as "difference in the standard deviation") between the standard deviation of each feature data in the groups $G_{\mu}$, $G_{\nu}$, and $G_{\rho}$ in the original population including only the known specimens and the standard deviation of each feature data in the groups $G_{\mu}$, $G_{\nu}$, and $G_{\rho}$ in the new population to which a new specimen is added. Here, the degree-of-association calculating unit 43 may calculate the difference in the standard deviation for only the standard deviation of the feature data selected beforehand from plural feature data. In this case, the operator may select any feature data, or the ultrasonic observation apparatus 1 may automatically select any feature data.

FIG. 18 is a view schematically illustrating the standard deviation of each group and the standard deviation of the new population, when the feature data space illustrated in FIG. 10 is used. In FIG. 18, the standard deviation of the intercept b is supposed to be $\sigma_{\mu 1}$, and the standard deviation of the intensity c is supposed to be $\sigma_{\mu 2}$ in the group $G_{\mu}$. The standard deviation of the intercept b is supposed to be $\sigma_{\nu 1}$, and the standard deviation of the intensity c is supposed to be $\sigma_{\nu 2}$ in the group $G_{\nu}$. The standard deviation of the intercept b is supposed to be $\sigma_{\rho 1}$, and the standard deviation of the intensity c is supposed to be $\sigma_{\rho 2}$ in the group $G_{\rho}$. On the other hand, the standard deviation of the intercept b is supposed to be $\sigma_{\mu 1}'$, and the standard deviation of the intensity c is supposed to be $\sigma_{\mu 2}'$ in the population formed by adding the specimen point Sp to the group $G_{\mu}$. The standard deviation of the intercept b is supposed to be $\sigma_{\nu 1}'$, and the standard deviation of the intensity c is supposed to be $\sigma_{\nu 2}'$ in the population formed by adding the specimen point Sp to the group $G_{\nu}$. The standard deviation of the intercept b is supposed to be $\sigma_{\rho 1}'$, and the standard deviation of the intensity c is supposed to be $\sigma_{\rho 2}'$ in the population formed by adding the specimen point Sp to the group $G_{\rho}$. In this case, the above-mentioned difference in the standard deviation is obtained such as $\Delta\sigma_{\mu 1} = \sigma_{\mu 1}' - \sigma_{\mu 1}$, $\Delta\sigma_{\mu 2} = \sigma_{\mu 2}' - \sigma_{\mu 2}$, $\Delta\sigma_{\nu 1} = \sigma_{\nu 1}' - \sigma_{\nu 1}$ ... When the variables in the RGB color system are used, the red component, the green component, and the blue component may respectively be assigned to the differences in the standard deviation $\Delta\sigma_{\mu 1}$, $\Delta\sigma_{\nu 1}$, and $\Delta\sigma_{\rho 1}$ in the intercept b, for example. Alternatively, three attributes of light (color phase, brightness, saturation), which are different from the others, may be assigned to the differences in the standard deviation $\Delta\sigma_{\mu 1}$, $\Delta\sigma_{\nu 1}$, and $\Delta\sigma_{\rho 1}$. In this case, the attribute value is changed according to the difference in the standard deviation. A pattern determined according to the distance may be assigned to any one of the differences in the standard deviation $\Delta\sigma_{\mu 1}$, $\Delta\sigma_{\nu 1}$, and $\Delta\sigma_{\rho 1}$.

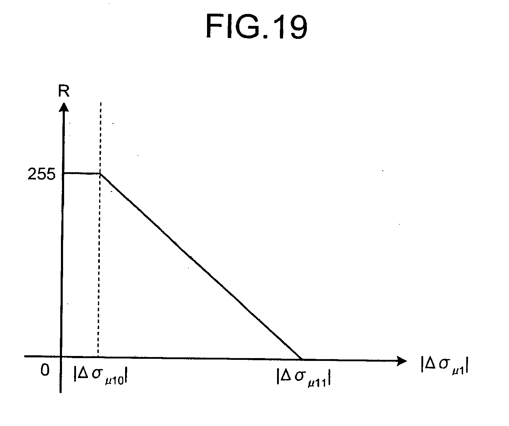

FIG. 19 is a view illustrating one example of the relationship between the absolute value $|\Delta\sigma_{\mu 1}| = |\sigma_{\mu 1}' - \sigma_{\mu 1}|$ and the magnitude of the red component R. In FIG. 19, when the absolute value $|\Delta\sigma_{\mu 1}|$ satisfies $0 \le |\Delta\sigma_{\mu 1}| \le |\Delta\sigma_{\mu 10}|$, the magnitude of the red component R is 255 (8-bit notation). When the absolute value $|\Delta\sigma_{\mu 1}|$ satisfies $|\Delta\sigma_{\mu 10}| < |\Delta\sigma_{\mu 1}| \le |\Delta\sigma_{\mu 11}|$, the magnitude of the red component R linearly decreases, and when the absolute value $|\Delta\sigma_{\mu 1}|$ satisfies $|\Delta\sigma_{\mu 1}| > |\Delta\sigma_{\mu 11}|$, it is zero. Even in the third embodiment, the magnitude of the color component assigned to each pixel in the calculation result display image data may arbitrarily be set.

The degree-of-association calculating unit 43 may calculate a value, which is obtained by weighting to the difference in the standard deviation of all feature data for each group, and may determine the tissue characterization corresponding to the group having the minimum value as the tissue characterization of the specimen. In this case, when the feature data has the slope a, the intercept b, and the intensity c, the degree-of-association calculating unit 43 calculates $w_a \cdot$(the difference in the standard deviation of a) + $w_b \cdot$(the difference in the standard deviation of b) + $w_c \cdot$(the difference in the standard deviation of c) by setting the weights respectively corresponding to the slope a, the intercept b, and the intensity c as $w_a$, $w_b$, and $w_c$, and determines the tissue characterization of the specimen based upon the calculated value. The operator may arbitrarily set the weights $w_a$, $w_b$, and $w_c$, or the ultrasonic observation apparatus 1 may automatically set the weights $w_a$, $w_b$, and $w_c$.

The degree-of-association calculating unit 43 may calculate a square root of a value obtained by performing the weighting to the square of the difference in the standard deviation of all feature data for each group, and may determine the tissue characterization corresponding to the group having the minimum square root as the tissue characterization of the specimen. In this case, when the feature data has the slope a, the intercept b, and the intensity c, the degree-of-association calculating unit 43 calculates $\{w'_a \cdot$(the difference in the standard deviation of a)$^2$ + $w'_b \cdot$(the difference in the standard deviation of b)$^2$ + $w'_c \cdot$(the difference in the standard deviation of c)$^2\}^{1/2}$ by setting the weights respectively corresponding to the slope a, the intercept b, and the intensity c as $w'_a$, $w'_b$, and $w'_c$, and determines the tissue characterization of the specimen based upon the calculated value. The operator may arbitrarily set the weights $w'_a$, $w'_b$, and $w'_c$, or the ultrasonic observation apparatus 1 may automatically set the weights $w'_a$, $w'_b$, and $w'_c$.

According to the third embodiment described above, the specimen can precisely be observed, whereby the reliability in the observation result can be enhanced, as in the first embodiment. According to the third embodiment, the influence of the attenuation caused by the propagation of the ultrasonic wave can be eliminated, whereby the tissue characterization can be distinguished with higher accuracy.

In the third embodiment, the degree-of-association calculating unit 43 determines the tissue characterization based upon the difference in the standard deviation of each feature data between the original population and the population formed by adding a new specimen. However, this is only one example. For example, the degree-of-association calculating unit 43 may determine the tissue characterization based upon the difference in the average of each feature data between the original population and the population formed by adding a new specimen.

The embodiments for carrying out the present invention have been described above. The present invention is not limited to the above-mentioned first to third embodiments. Specifically, the present invention can include various forms without departing from the technical scope described in the claims.

Reference Signs List

| 1 | ULTRASONIC OBSERVATION APPARATUS |
| --- | --- |
| 2 | ULTRASONIC PROBE |
| 3 | TRANSMISSION/RECEPTION UNIT |
| 4 | COMPUTATION UNIT |
| 5 | IMAGE PROCESSING UNIT |
| 6 | INPUT UNIT |
| 7 | DISPLAY UNIT |
| 8 | STORAGE UNIT |
| 9 | CONTROL UNIT |

21          SIGNAL CONVERSION UNIT

41          FREQUENCY ANALYZING UNIT

42          FEATURE DATA EXTRACTING UNIT

43          DEGREE-OF-ASSOCIATION CALCULATING UNIT

51          B-MODE IMAGE DATA GENERATING UNIT

52          CALCULATION RESULT DISPLAY IMAGE DATA GENERATING UNIT

81          KNOWN SPECIMEN INFORMATION STORAGE UNIT

82          WINDOW FUNCTION STORAGE UNIT

83          CORRECTION INFORMATION STORAGE UNIT

100         B-MODE IMAGE

300, 400, 500    CALCULATION RESULT DISPLAY IMAGE

300g        GREEN REGION

300r        RED REGION

421         APPROXIMATION UNIT

422         ATTENUATION CORRECTION UNIT

**Claims**

1. An ultrasonic observation apparatus (1) configured to transmit an ultrasonic wave to a specimen, and receive the ultrasonic wave reflected on the specimen, comprising:

    a frequency analyzing unit (41) configured to calculate a frequency spectrum by analyzing a frequency of the received ultrasonic wave;
    a feature data extracting unit (42) configured to extract feature data of the frequency spectrum by performing an approximation to the frequency spectrum calculated by the frequency analyzing unit (41);
    a storage unit (8) configured to store feature data of a frequency spectrum extracted based upon the ultrasonic wave reflected respectively from plural known specimens, and to store the plural known specimens as being classified into plural groups based upon information of each of the known specimens;
    a degree-of-association calculating unit (43) configured to calculate a degree of association between the feature data extracted by the feature data extracting unit (42) and each group by using a statistics of a population including at least the feature data of each group stored in the storage unit (8);
    an image processing unit (5) configured to allow the degree of association of the specimen to each group to correspond to different color components, and to generate ultrasonic image data by using a parameter formed by combining the different color components, based upon the calculation result of the degree-of-association calculating unit (43); and
    a display unit (7) configured to be capable of displaying the image corresponding to the ultrasonic image data generated by the image processing unit (5),
    **characterized in that** the feature data extracting unit (42) includes
    an approximation unit (421) configured to extract pre-correction feature data, which is an amount before an attenuation correction process for reducing a contribution of an attenuation caused according to a reception depth and a frequency of the ultrasonic wave during the propagation of the ultrasonic wave to the frequency spectrum is executed, through an execution of an approximation process to the frequency spectrum calculated by the frequency analyzing unit (41); and

an attenuation correction unit (422) configured to extract the feature data of the frequency spectrum by performing the attenuation correction process to the pre-correction feature data extracted by the approximation unit (421).

2. The ultrasonic observation apparatus (1) according to claim 1, wherein
the feature data extracting unit (42) extracts plural feature data,
the storage unit (8) stores an average of the respective feature data in each of the plural groups,
the degree-of-association calculating unit (43) sets feature data space having at least any one of the plural feature data as a component, and calculates, as the degree of association, a distance on the feature data space between a specimen point that has a feature data, which serves as the component of the feature data space, out of the feature data of the frequency spectrum of the specimen as a coordinate on the feature data space, and a known specimen average point that has an average of the feature data, which serve as the component of the feature data space, out of the feature data in the group of the plural known specimens as a coordinate on the feature data space.

3. The ultrasonic observation apparatus (1) according to claim 2, wherein
the image processing unit (5) determines a pixel value of the ultrasonic image data according to the distance between the specimen point and the known specimen average point.

4. The ultrasonic observation apparatus (1) according to claim 1, wherein
the degree-of-association calculating unit (43) calculates a standard deviation of a feature data in a new population formed by adding a feature data of the specimen to the feature data belonging to the same group, and calculates the difference between the standard deviation and the standard deviation of the feature data belonging to the same group as the degree of association.

5. The ultrasonic observation apparatus (1) according to claim 4, wherein
the image processing unit (5) determines the pixel value of the ultrasonic image data according to the difference between the standard deviation of the feature data in the new population and the standard deviation of the feature data belonging to the same group.

6. The ultrasonic observation apparatus (1) according to claim 1, wherein
the plural specimens having the feature data belonging to the same group have the same tissue characterization.

7. The ultrasonic observation apparatus (1) according to claim 1, wherein
the approximation unit (421) approximates the frequency spectrum with a polynomial according to a regression analysis.

8. The ultrasonic observation apparatus (1) according to claim 7, wherein
the approximation unit (421) approximates the frequency spectrum with a primary expression, and
extracts plural feature data including at least two of a slope of the primary expression, an intercept of the primary expression, and an intensity determined by using the slope, the intercept, and a specific frequency included in a frequency band of the frequency spectrum.

9. The ultrasonic observation apparatus (1) according to claim 1, wherein
the attenuation correction unit (422) makes a great correction, as the reception depth of the ultrasonic wave is larger.

10. The ultrasonic observation apparatus (1) according to claim 1, wherein
the feature data extracting unit (42) includes:

an attenuation correction unit (422) configured to execute an attenuation correction process for reducing a contribution of an attenuation caused according to a reception depth and a frequency of the ultrasonic wave during the propagation of the ultrasonic wave to the frequency spectrum; and
an approximation unit (421) configured to extract the feature data of the frequency spectrum by performing the approximation process to the frequency spectrum that is corrected by the attenuation correction unit (422).

11. The ultrasonic observation apparatus (1) according to claim 10, wherein
the approximation unit (421) approximates the frequency spectrum with a polynomial according to a regression analysis.

12. The ultrasonic observation apparatus (1) according to claim 11, wherein

the approximation unit (421) approximates the frequency spectrum with a primary expression, and extracts plural feature data including at least two of a slope of the primary expression, an intercept of the primary expression, and an intensity determined by using the slope, the intercept, and a specific frequency included in a frequency band of the frequency spectrum.

13. The ultrasonic observation apparatus (1) according to claim 10, wherein the attenuation correction unit (422) makes a great correction, as the reception depth of the ultrasonic wave is larger.

14. An operation method of an ultrasonic observation apparatus (1) that transmits an ultrasonic wave to a specimen, and receives the ultrasonic wave reflected on the specimen, comprising:

a frequency analyzing step including calculating a frequency spectrum by analyzing a frequency of the received ultrasonic wave by a frequency analyzing unit (41);
a feature data extracting step including extracting feature data of the frequency spectrum by performing an approximation to the frequency spectrum calculated at the frequency analyzing step, by a feature data extracting unit (42);
a degree-of-association calculating step including reading a feature data of each group from a storage unit (8) that stores feature data of a frequency spectrum extracted based upon the ultrasonic wave reflected respectively from plural known specimens and that stores the plural known specimens as being classified into plural groups based upon information of each of the known specimens, and including calculating a degree of association between the feature data extracted by the feature data extracting unit and each group by using a statistics of a population including at least the feature data of each group, by a degree-of-association calculating unit (43);
an image processing step including allowing the degree of association of the specimen to each group to correspond to different color components and including generating ultrasonic image data by using a parameter formed by combining the different color components, based upon the calculation result obtained at the degree-of-association calculating step, by an image processing unit (5); and
an image display step including displaying the image corresponding to the ultrasonic image data generated at the image processing step on a display unit (7),
**characterized by**
extracting, by an approximation unit (421), pre-correction feature data, which is an amount before an attenuation correction process for reducing a contribution of an attenuation caused according to a reception depth and a frequency of the ultrasonic wave during the propagation of the ultrasonic wave to the frequency spectrum is executed, through an execution of an approximation process to the frequency spectrum calculated by the frequency analyzing unit (41); and
extracting, by an attenuation correction unit (422), the feature data of the frequency spectrum by performing the attenuation correction process to the pre-correction feature data extracted by the approximation unit (421).

15. An operating program adapted to operate an ultrasonic observation apparatus (1) that transmits an ultrasonic wave to a specimen, and receives the ultrasonic wave reflected on the specimen, wherein the operating program instructs the ultrasonic observation apparatus (1) to perform:

a frequency analyzing step including calculating a frequency spectrum by analyzing a frequency of the received ultrasonic wave by a frequency analyzing unit (41);
a feature data extracting step including extracting feature data of the frequency spectrum by performing an approximation to the frequency spectrum calculated at the frequency analyzing step, by a feature data extracting unit (42);
a degree-of-association calculating step including reading a feature data of each group from a storage unit that stores feature data of a frequency spectrum extracted based upon the ultrasonic wave reflected respectively from plural known specimens and that stores the plural known specimens as being classified into plural groups based upon information of each of the known specimens, and including calculating a degree of association between the feature data extracted by the feature data extracting unit (42) and each group by using a statistics of a population including at least the feature data of each group, by a degree-of-association calculating unit (43);
an image processing step including allowing the degree of association of the specimen to each group to correspond to different color components and including generating ultrasonic image data by using a parameter formed by combining the different color components, based upon the calculation result obtained at the degree-of-association calculating step, by an image processing unit (5); and
an image display step including displaying the image corresponding to the ultrasonic image data generated at the image processing step on a display unit (7),

**characterized by**

extracting, by an approximation unit (421), pre-correction feature data, which is an amount before an attenuation correction process for reducing a contribution of an attenuation caused according to a reception depth and a frequency of the ultrasonic wave during the propagation of the ultrasonic wave to the frequency spectrum is executed, through an execution of an approximation process to the frequency spectrum calculated by the frequency analyzing unit (41); and

extracting, by an attenuation correction unit (422), the feature data of the frequency spectrum by performing the attenuation correction process to the pre-correction feature data extracted by the approximation unit (421).

**Patentansprüche**

1. Ultraschallbeobachtungsvorrichtung (1), die dazu ausgebildet ist, eine Ultraschallwelle zu einer Probe zu senden und die von der Probe reflektierte Ultraschallwelle zu empfangen, mit:

einer Frequenzanalyseeinheit (41), die dazu ausgebildet ist, ein Frequenzspektrum durch Analyse einer Frequenz der empfangenen Ultraschallwelle zu berechnen;

eine Merkmalsdaten-Extraktionseinheit (42), die zum Extrahieren von Merkmalsdaten des Frequenzspektrums ausgebildet ist, indem eine Näherung bezüglich des durch die Frequenzanalyseeinheit (41) berechneten Frequenzspektrums ausgeführt wird;

eine Speichereinheit (8), die dazu ausgebildet ist, Merkmalsdaten eines Frequenzspektrums zu speichern, die basierend auf der jeweils von mehreren bekannten Proben reflektierten Ultraschallwelle extrahiert wurden, und die mehreren bekannten Proben als in mehrere Gruppen klassifiziert basierend auf Information einer jeden der bekannten Proben zu speichern;

eine Einheit (43) zum Berechnen des Zugehörigkeitsgrads, die dazu ausgebildet ist, einen Zugehörigkeitsgrad zwischen den von der Merkmalsdaten-Extraktionseinheit (42) extrahierten Merkmalsdaten und jeder Gruppe unter Verwendung einer Populationsstatistik mit wenigstens den Merkmalsdaten einer jeden in der Speichereinheit (8) gespeicherten Gruppe zu berechnen;

eine Bildverarbeitungseinheit (5), die dazu ausgebildet ist, es zu ermöglichen, dass der Zugehörigkeitsgrad der Probe zu jeder Gruppe verschiedenen Farbkomponenten entspricht, und basierend auf dem Berechnungsergebnis der Einheit (43) zum Berechnen des Zugehörigkeitsgrads Ultraschallbilddaten unter Verwendung eines Parameters zu generieren, der durch Kombination der verschiedenen Farbkomponenten gebildet wird; und

eine Anzeigeeinheit (7), die so ausgebildet ist, dass sie das Bild, das den von der Bildverarbeitungseinheit (5) generierten Ultraschallbilddaten entspricht, anzuzeigen vermag,

**dadurch gekennzeichnet, dass** die Merkmalsdaten-Extraktionseinheit (42) enthält

eine Näherungseinheit (421), die dazu ausgebildet ist, durch Ausführung eines Näherungsprozesses bezüglich des durch die Frequenzanalyseeinheit (41) berechnete Frequenzspektrums Vorkorrektur-Merkmalsdaten zu extrahieren, die einen Betrag darstellen, bevor zur Reduzierung eines gemäß einer Eindringtiefe und einer Frequenz der Ultraschallwelle während der Ausbreitung der Ultraschallwelle verursachten Dämpfungsbeitrags ein Dämpfungskorrekturprozess bezüglich des Frequenzspektrums ausgeführt wird; und

eine Dämpfungskorrektureinheit (422), die dazu ausgebildet ist, die Merkmalsdaten des Frequenzspektrums zu extrahieren, indem der Dämpfungskorrekturprozess für die von der Näherungseinheit (421) extrahierten Vorkorrektur-Merkmalsdaten ausgeführt wird.

2. Ultraschallbeobachtungsvorrichtung (1) nach Anspruch 1, wobei

die Merkmalsdaten-Extraktionseinheit (42) mehrere Merkmalsdaten extrahiert,

die Speichereinheit (8) einen Durchschnitt der jeweiligen Merkmalsdaten in jeder der mehreren Gruppen speichert,

die Einheit (43) zum Berechnen des Zugehörigkeitsgrads einen Merkmalsdatenraum mit wenigstens einer beliebigen von den mehreren Merkmalsdaten als eine Komponente festlegt und als Zugehörigkeitsgrad einen Abstand im Merkmalsdatenraum zwischen einem Probenpunkt mit Merkmalsdaten, die als Komponente des Merkmalsdatenraums aus den Merkmalsdaten des Frequenzspektrums der Probe als eine Koordinate im Merkmalsdatenraum dienen, und einem bekannten Durchschnittsprobenpunkt mit einem Durchschnitt der Merkmalsdaten, die als Komponente des Merkmalsdatenraums aus den Merkmalsdaten in der Gruppe der mehreren bekannten Proben als eine Koordinate im Merkmalsdatenraum dienen, berechnet.

3. Ultraschallbeobachtungsvorrichtung (1) nach Anspruch 2, wobei

die Bildverarbeitungseinheit (5) einen Pixelwert der Ultraschallbilddaten gemäß dem Abstand zwischen dem Probenpunkt und dem bekannten Durchschnittsprobenpunkt bestimmt.

**4.** Ultraschallbeobachtungsvorrichtung (1) nach Anspruch 1, wobei
die Einheit (43) zum Berechnen des Zugehörigkeitsgrads eine Standardabweichung eines Merkmalsdatenwertes in einer neuen Population berechnet, die durch Hinzufügen eines Merkmalsdatenwertes der Probe zu den zur gleichen Gruppe gehörenden Merkmalsdaten gebildet wird, und die Differenz zwischen der Standardabweichung und der Standardabweichung der zur gleichen Gruppe gehörenden Merkmalsdaten als Zugehörigkeitsgrad berechnet.

**5.** Ultraschallbeobachtungsvorrichtung (1) nach Anspruch 4, wobei
die Bildverarbeitungseinheit (5) den Pixelwert der Ultraschallbilddaten gemäß der Differenz zwischen der Standardabweichung der Merkmalsdaten in der neuen Population und der Standardabweichung der zur gleichen Gruppe gehörenden Merkmalsdaten bestimmt.

**6.** Ultraschallbeobachtungsvorrichtung (1) nach Anspruch 1, wobei
die mehreren Proben mit den zur gleichen Gruppe gehörenden Merkmalsdaten die gleiche Gewebecharakterisierung aufweisen.

**7.** Ultraschallbeobachtungsvorrichtung (1) nach Anspruch 1, wobei
die Näherungseinheit (421) die Näherung bezüglich des Frequenzspektrums mit einem Polynom gemäß einer Regressionsanalyse ausführt.

**8.** Ultraschallbeobachtungsvorrichtung (1) nach Anspruch 7, wobei
die Näherungseinheit (421) die Näherung bezüglich des Frequenzspektrums mit einem primären Ausdruck ausführt, und
mehrere Merkmalsdaten extrahiert, darunter wenigstens zwei von einer Steigung des primären Ausdrucks, eines Abschnitts des primären Ausdrucks und einer Intensität, die unter Verwendung der Steigung, des Abschnitts und einer in einem Frequenzband des Frequenzspektrums enthaltenen speziellen Frequenz bestimmt wurde.

**9.** Ultraschallbeobachtungsvorrichtung (1) nach Anspruch 1, wobei
die Dämpfungskorrektureinheit (422) eine große Korrektur ausführt, wenn die Eindringtiefe der Ultraschallwelle größer ist.

**10.** Ultraschallbeobachtungsvorrichtung (1) nach Anspruch 1, wobei
die Merkmalsdaten-Extraktionseinheit (42) beinhaltet:

eine Dämpfungskorrektureinheit (422), die dazu ausgebildet ist, einen Dämpfungskorrekturprozess zur Reduzierung eines gemäß einer Eindringtiefe und einer Frequenz der Ultraschallwelle während der Ausbreitung der Ultraschallwelle verursachten Dämpfungsbeitrags für das Frequenzspektrum auszuführen; und
eine Näherungseinheit (421), die dazu ausgebildet ist, die Merkmalsdaten des Frequenzspektrums durch Durchführung des Näherungsprozesses für das durch die Dämpfungskorrektureinheit (422) korrigierte Frequenzspektrum zu extrahieren.

**11.** Ultraschallbeobachtungsvorrichtung (1) nach Anspruch 10, wobei
die Näherungseinheit (421) die Näherung bezüglich des Frequenzspektrums mit einem Polynom gemäß einer Regressionsanalyse ausführt.

**12.** Ultraschallbeobachtungsvorrichtung (1) nach Anspruch 11, wobei
die Näherungseinheit (421) die Näherung bezüglich des Frequenzspektrums mit einem primären Ausdruck ausführt, und
mehrere Merkmalsdaten extrahiert, darunter wenigstens zwei von einer Steigung des primären Ausdrucks, eines Abschnitts des primären Ausdrucks und einer Intensität, die unter Verwendung der Steigung, des Abschnitts und einer in einem Frequenzband des Frequenzspektrums enthaltenen speziellen Frequenz bestimmt wurde.

**13.** Ultraschallbeobachtungsvorrichtung (1) nach Anspruch 10, wobei
die Dämpfungskorrektureinheit (422) eine große Korrektur ausführt, wenn die Eindringtiefe der Ultraschallwelle größer ist.

**14.** Verfahren zum Betreiben einer Ultraschallbeobachtungsvorrichtung (1), die eine Ultraschallwelle zu einer Probe sendet und die von der Probe reflektierte Ultraschallwelle empfängt, wobei das Verfahren aufweist:

einen Frequenzanalyseschritt, der die Berechnung eines Frequenzspektrums durch Analyse einer Frequenz der empfangenen Ultraschallwelle durch eine Frequenzanalyseeinheit (41) beinhaltet;

einen Merkmalsdaten-Extraktionsschritt, der das Extrahieren von Merkmalsdaten des Frequenzspektrums durch Ausführen einer Näherung bezüglich des im Frequenzanalyseschritt berechneten Frequenzspektrums durch eine Merkmalsdaten-Extraktionseinheit (42) beinhaltet;

einen Schritt zur Berechnung des Zugehörigkeitsgrades, der das Lesen eines Merkmalsdatenwertes von einer jeden Gruppe aus einer Speichereinheit (8) beinhaltet, die Merkmalsdaten eines Frequenzspektrums speichert, die basierend auf der jeweils von mehreren bekannten Proben reflektierten Ultraschallwelle extrahiert werden, und die mehreren bekannten Proben als in mehrere Gruppen klassifiziert basierend auf Informationen einer jeden der bekannten Proben speichert, und die Berechnung eines Zugehörigkeitsgrads zwischen den von der Merkmalsdaten-Extraktionseinheit extrahierten Merkmalsdaten und jeder Gruppe unter Verwendung einer Populationsstatistik mit wenigstens den Merkmalsdaten einer jeder Gruppe durch eine Einheit (43) zum Berechnen des Zugehörigkeitsgrads beinhaltet;

einen Bildverarbeitungsschritt, bei dem es ermöglicht wird, dass der Zugehörigkeitsgrad der Proben zu jeder Gruppe verschiedenen Farbkomponenten entspricht, und bei dem durch eine Bildverarbeitungseinheit (5) Ultraschallbilddaten unter Verwendung eines Parameters generiert werden, der durch Kombination der verschiedenen Farbkomponenten basierend auf dem im Berechnungsschritt des Zugehörigkeitsgrads erhaltenen Berechnungsergebnis gebildet wird; und

einen Bildanzeigeschritt, der die Anzeige des Bildes entsprechend den im Bildverarbeitungsschritt generierten Ultraschallbilddaten auf einer Anzeigeeinheit (7) beinhaltet,

**dadurch gekennzeichnet, dass**

durch eine Näherungseinheit (421) Vorkorrektur-Merkmalsdaten extrahiert werden, die einen Betrag darstellen, bevor zur Reduzierung eines gemäß einer Eindringtiefe und einer Frequenz der Ultraschallwelle während der Ausbreitung der Ultraschallwelle verursachten Dämpfungsbeitrags ein Dämpfungskorrekturprozess bezüglich des Frequenzspektrums ausgeführt wird, durch Ausführung eines Näherungsprozesses bezüglich des durch die Frequenzanalyseeinheit (41) berechneten Frequenzspektrums; und dass

durch eine Dämpfungskorrektureinheit (422) Merkmalsdaten des Frequenzspektrums extrahiert werden, indem der Dämpfungskorrekturprozess für die durch die Näherungseinheit (421) extrahierten Vorkorrektur-Merkmalsdaten ausgeführt wird.

15. Betriebsprogramm, das dazu ausgebildet ist, eine Ultraschallbeobachtungsvorrichtung (1) zu betreiben, die eine Ultraschallwelle zu einer Probe sendet und die von der Probe reflektierte Ultraschallwelle empfängt, wobei das Betriebsprogramm die Ultraschallbeobachtungsvorrichtung (1) dazu anweist, die folgenden Schritte auszuführen:

einen Frequenzanalyseschritt, der die Berechnung eines Frequenzspektrums durch Analyse einer Frequenz der empfangenen Ultraschallwelle durch eine Frequenzanalyseeinheit (41) beinhaltet;

einen Merkmalsdaten-Extraktionsschritt, der das Extrahieren von Merkmalsdaten des Frequenzspektrums durch Ausführen einer Näherung bezüglich des im Frequenzanalyseschritt berechneten Frequenzspektrums durch eine Merkmalsdaten-Extraktionseinheit (42) beinhaltet;

einen Schritt zur Berechnung des Zugehörigkeitsgrades, der das Lesen eines Merkmalsdatenwertes von einer jeden Gruppe aus einer Speichereinheit beinhaltet, die Merkmalsdaten eines Frequenzspektrums speichert, die basierend auf der jeweils von mehreren bekannten Proben reflektierten Ultraschallwelle extrahiert werden, und die mehreren bekannten Proben als in mehrere Gruppen klassifiziert basierend auf Informationen einer jeden der bekannten Proben speichert, und die Berechnung eines Zugehörigkeitsgrads zwischen den von der Merkmalsdaten-Extraktionseinheit (42) extrahierten Merkmalsdaten und jeder Gruppe unter Verwendung einer Populationsstatistik mit wenigstens den Merkmalsdaten einer jeder Gruppe durch eine Einheit (43) zum Berechnen des Zugehörigkeitsgrads beinhaltet;

einen Bildverarbeitungsschritt, bei dem es ermöglicht wird, dass der Zugehörigkeitsgrad der Proben zu jeder Gruppe verschiedenen Farbkomponenten entspricht, und bei dem durch eine Bildverarbeitungseinheit (5) Ultraschallbilddaten unter Verwendung eines Parameters generiert werden, der durch Kombination der verschiedenen Farbkomponenten basierend auf dem im Berechnungsschritt des Zugehörigkeitsgrads erhaltenen Berechnungsergebnis gebildet wird; und

einen Bildanzeigeschritt, der die Anzeige des Bildes entsprechend den im Bildverarbeitungsschritt generierten Ultraschallbilddaten auf einer Anzeigeeinheit (7) beinhaltet,

**dadurch gekennzeichnet, dass**

durch eine Näherungseinheit (421) Vorkorrektur-Merkmalsdaten extrahiert werden, die einen Betrag darstellen, bevor zur Reduzierung eines gemäß einer Eindringtiefe und einer Frequenz der Ultraschallwelle während der Ausbreitung der Ultraschallwelle verursachten Dämpfungsbeitrags ein Dämpfungskorrekturprozess bezüglich des Frequenzspektrums ausgeführt wird, durch Ausführung eines Näherungsprozesses bezüglich des durch die Frequenzanalyseeinheit (41) berechneten Frequenzspektrums; und dass durch eine Dämpfungskorrektureinheit (422) Merkmalsdaten des Frequenzspektrums extrahiert werden, indem der Dämpfungskorrekturprozess für die durch die Näherungseinheit (421) extrahierten Vorkorrektur-Merkmalsdaten ausgeführt wird.

## Revendications

1. Appareil d'observation ultrasonore (1) configuré pour transmettre une onde ultrasonore jusqu'à un spécimen, et recevoir l'onde ultrasonore réfléchie sur le spécimen, comprenant :

une unité (41) d'analyse de fréquence configurée pour calculer un spectre de fréquence en analysant une fréquence de l'onde ultrasonore reçue ;
une unité (42) d'extraction de données de caractéristiques configurée pour extraire des données de caractéristiques du spectre de fréquence en effectuant une approximation du spectre de fréquence calculé par l'unité (41) d'analyse de fréquence ;
une unité (8) de stockage configurée pour stocker des données de caractéristiques d'un spectre de fréquence extraites sur la base de l'onde ultrasonore réfléchie respectivement de spécimens connus multiples, et pour stocker les spécimens connus multiples comme étant classés en plusieurs groupes sur la base d'une information de chacun des spécimens connus ;
une unité (43) de calcul de degré d'association configurée pour calculer un degré d'association entre les données de caractéristiques extraites par l'unité (42) d'extraction de données de caractéristiques et chaque groupe en utilisant une statistique d'une population incluant au moins les données de caractéristiques de chaque groupe stocké dans l'unité (8) de stockage ;
une unité (5) de traitement d'image configurée pour permettre que le degré d'association du spécimen à chaque groupe corresponde à différentes composantes de couleur, et pour générer des données d'image ultrasonore en utilisant un paramètre formé en combinant les différentes composantes de couleur, sur la base du résultat de calcul de l'unité (43) de calcul de degré d'association ; et
une unité (7) d'affichage configurée pour être apte à afficher l'image correspondant aux données d'image ultrasonore générées par l'unité (5) de traitement d'image,
**caractérisé en ce que** l'unité (42) d'extraction de données de caractéristiques inclut
une unité (421) d'approximation configurée pour extraire des données de caractéristiques précorrection, qui sont une quantité avant un processus de correction d'atténuation pour réduire une contribution d'une atténuation causée en fonction d'une profondeur de réception et d'une fréquence de l'onde ultrasonore pendant que la propagation de l'onde ultrasonore sur le spectre de fréquence est exécutée, par l'intermédiaire d'une exécution d'un processus d'approximation sur le spectre de fréquence calculé par l'unité (41) d'analyse de fréquence ; et
une unité (422) de correction d'atténuation configurée pour extraire les données de caractéristiques du spectre de fréquence en exécutant le processus de correction d'atténuation sur les données de caractéristiques précorrection extraites par l'unité (421) d'approximation.

2. Appareil d'observation ultrasonore (1) selon la revendication 1, dans lequel
l'unité (42) d'extraction de données de caractéristiques extrait des données de caractéristiques multiples,
l'unité (8) de stockage stocke une moyenne des données de caractéristiques respectives dans chacun des groupes multiples,
l'unité (43) de calcul de degré d'association définit un espace de données de caractéristiques ayant au moins l'une quelconque parmi les données de caractéristiques multiples comme une composante, et calcule, comme le degré d'association, une distance sur l'espace de données de caractéristiques entre un point de spécimen qui a une donnée de caractéristique, qui sert comme la composante de l'espace de données de caractéristiques, parmi les données de caractéristiques du spectre de fréquence du spécimen comme une coordonnée sur l'espace de données de caractéristiques, et un point moyen de spécimens connus qui a une moyenne des données de caractéristiques, qui sert comme la composante de l'espace de données de caractéristiques, parmi les données de caractéristiques dans le groupe des spécimens connus multiples comme une coordonnée sur l'espace de données de caractéristiques.

3. Appareil d'observation ultrasonore (1) selon la revendication 2, dans lequel
l'unité (5) de traitement d'image détermine une valeur de pixel des données d'image ultrasonore en fonction de la distance entre le point de spécimen et le point moyen de spécimens connus.

**4.** Appareil d'observation ultrasonore (1) selon la revendication 1, dans lequel
l'unité (43) de calcul de degré d'association calcule un écart standard d'une donnée de caractéristique dans une nouvelle population formée en ajoutant une donnée de caractéristique du spécimen aux données de caractéristiques appartenant au même groupe, et calcule la différence entre l'écart standard et l'écart standard des données de caractéristiques appartenant au même groupe comme le degré d'association.

**5.** Appareil d'observation ultrasonore (1) selon la revendication 4, dans lequel
l'unité (5) de traitement d'image détermine la valeur de pixel des données d'image ultrasonore en fonction de la différence entre l'écart standard des données de caractéristiques dans la nouvelle population et l'écart standard des données de caractéristiques appartenant au même groupe.

**6.** Appareil d'observation ultrasonore (1) selon la revendication 1, dans lequel
les spécimens multiples ayant les données de caractéristiques appartenant au même groupe ont la même caractérisation de tissu.

**7.** Appareil d'observation ultrasonore (1) selon la revendication 1, dans lequel
l'unité (421) d'approximation approche le spectre de fréquence avec un polynôme conformément à une analyse de régression.

**8.** Appareil d'observation ultrasonore (1) selon la revendication 7, dans lequel
l'unité (421) d'approximation approche le spectre de fréquence avec une expression primaire, et
extrait des données de caractéristiques multiples incluant au moins deux parmi une pente de l'expression primaire, un point d'intersection de l'expression primaire, et une intensité déterminée en utilisant la pente, le point d'intersection, et une fréquence spécifique incluse dans une bande de fréquence du spectre de fréquence.

**9.** Appareil d'observation ultrasonore (1) selon la revendication 1, dans lequel
l'unité (422) de correction d'atténuation apporte une correction d'autant plus importante que la profondeur de réception de l'onde ultrasonore est grande.

**10.** Appareil d'observation ultrasonore (1) selon la revendication 1, dans lequel
l'unité (42) d'extraction de données de caractéristiques inclut :

une unité (422) de correction d'atténuation configurée pour exécuter un processus de correction d'atténuation pour réduire une contribution d'une atténuation causée en fonction d'une profondeur de réception et d'une fréquence de l'onde ultrasonore lors de la propagation de l'onde ultrasonore sur le spectre de fréquence ; et une unité (421) d'approximation configurée pour extraire les données de caractéristiques du spectre de fréquence en exécutant le processus d'approximation sur le spectre de fréquence qui est corrigé par l'unité (422) de correction d'atténuation.

**11.** Appareil d'observation ultrasonore (1) selon la revendication 10, dans lequel
l'unité (421) d'approximation approche le spectre de fréquence avec un polynôme conformément à une analyse de régression.

**12.** Appareil d'observation ultrasonore (1) selon la revendication 11, dans lequel
l'unité (421) d'approximation approche le spectre de fréquence avec une expression primaire, et
extrait des données de caractéristiques multiples incluant au moins deux parmi une pente de l'expression primaire, un point d'intersection de l'expression primaire, et une intensité déterminée en utilisant la pente, le point d'intersection, et une fréquence spécifique incluse dans une bande de fréquence du spectre de fréquence.

**13.** Appareil d'observation ultrasonore (1) selon la revendication 10, dans lequel
l'unité (422) de correction d'atténuation apporte une correction d'autant plus importante que la profondeur de réception de l'onde ultrasonore est grande.

**14.** Procédé de fonctionnement d'un appareil d'observation ultrasonore (1) qui transmet une onde ultrasonore jusqu'à un spécimen, et reçoit l'onde ultrasonore réfléchie sur le spécimen, comprenant :

une étape d'analyse de fréquence incluant le calcul d'un spectre de fréquence en analysant une fréquence de l'onde ultrasonore reçue par une unité (41) d'analyse de fréquence ;

une étape d'extraction de données de caractéristiques incluant l'extraction de données de caractéristiques du spectre de fréquence en exécutant une approximation du spectre de fréquence calculé à l'étape d'analyse de fréquence, par une unité (42) d'extraction de données de caractéristiques ;

une étape de calcul de degré d'association incluant la lecture d'une donnée de caractéristiques de chaque groupe dans une unité (8) de stockage qui stocke des données de caractéristiques d'un spectre de fréquence extrait sur la base de l'onde ultrasonore réfléchie respectivement de spécimens connus multiples et qui stocke les spécimens connus multiples comme étant classés en groupes multiples sur la base d'une information de chacun des spécimens connus, et incluant le calcul d'un degré d'association entre les données de caractéristiques extraites par l'unité d'extraction de données de caractéristiques et chaque groupe en utilisant une statistique d'une population incluant au moins les données de caractéristiques de chaque groupe, par une unité (43) de calcul de degré d'association ;

une étape de traitement d'image incluant de permettre que le degré d'association du spécimen à chaque groupe corresponde à différentes composantes de couleur, et incluant la génération de données d'image ultrasonore en utilisant un paramètre formé en combinant les différentes composantes de couleur, sur la base du résultat de calcul obtenu à l'étape de calcul de degré d'association, par une unité (5) de traitement d'image ; et

une étape d'affichage d'image incluant l'affichage de l'image correspondant aux données d'image ultrasonore générées à l'étape de traitement d'image par une unité (7) d'affichage,

**caractérisé par**

l'extraction, par une unité (421) d'approximation, de données de caractéristiques précorrection, qui sont une quantité avant un processus de correction d'atténuation pour réduire une contribution d'une atténuation causée en fonction d'une profondeur de réception et d'une fréquence de l'onde ultrasonore pendant que la propagation de l'onde ultrasonore au spectre de fréquence est exécutée, par l'intermédiaire d'une exécution d'un processus d'approximation sur le spectre de fréquence calculé par l'unité (41) d'analyse de fréquence ; et

l'extraction, par une unité (422) de correction d'atténuation, des données de caractéristiques du spectre de fréquence en exécutant le processus de correction d'atténuation sur les données de caractéristiques précorrection extraites par l'unité (421) d'approximation.

**15.** Programme de fonctionnement adapté à faire fonctionner un appareil d'observation ultrasonore (1) qui transmet une onde ultrasonore jusqu'à un spécimen, et reçoit l'onde ultrasonore réfléchie sur le spécimen, dans lequel le programme de fonctionnement indique à l'appareil d'observation ultrasonore (1) de mettre en oeuvre :

une étape d'analyse de fréquence incluant le calcul d'un spectre de fréquence en analysant une fréquence de l'onde ultrasonore reçue par une unité (41) d'analyse de fréquence ;

une étape d'extraction de données de caractéristiques incluant l'extraction de données de caractéristiques du spectre de fréquence en exécutant une approximation du spectre de fréquence calculé à l'étape d'analyse de fréquence, par une unité (42) d'extraction de données de caractéristiques ;

une étape de calcul de degré d'association incluant la lecture d'une donnée de caractéristiques de chaque groupe dans une unité (8) de stockage qui stocke des données de caractéristiques d'un spectre de fréquence extrait sur la base de l'onde ultrasonore réfléchie respectivement de spécimens connus multiples et qui stocke les spécimens connus multiples comme étant classés en groupes multiples sur la base d'une information de chacun des spécimens connus, et incluant le calcul d'un degré d'association entre les données de caractéristiques extraites par l'unité d'extraction de données de caractéristiques et chaque groupe en utilisant une statistique d'une population incluant au moins les données de caractéristiques de chaque groupe, par une unité (43) de calcul de degré d'association ;

une étape de traitement d'image incluant de permettre que le degré d'association du spécimen à chaque groupe corresponde à différentes composantes de couleur, et incluant la génération de données d'image ultrasonore en utilisant un paramètre formé en combinant les différentes composantes de couleur, sur la base du résultat de calcul obtenu à l'étape de calcul de degré d'association, par une unité (5) de traitement d'image ; et

une étape d'affichage d'image incluant l'affichage de l'image correspondant aux données d'image ultrasonore générées à l'étape de traitement d'image par une unité (7) d'affichage,

**caractérisé par**

l'extraction, par une unité (421) d'approximation, de données de caractéristiques précorrection, qui est une quantité avant un processus de correction d'atténuation pour réduire une contribution d'une atténuation causée en fonction d'une profondeur de réception et d'une fréquence de l'onde ultrasonore pendant que la propagation de l'onde ultrasonore au spectre de fréquence est exécutée, par l'intermédiaire d'une exécution d'un processus d'approximation sur le spectre de fréquence calculé par l'unité (41) d'analyse de fréquence ; et

l'extraction, par une unité (422) de correction d'atténuation, des données de caractéristiques du spectre de fréquence en exécutant le processus de correction d'atténuation sur les données de caractéristiques précorrection extraites par l'unité (421) d'approximation.

# FIG.1

# FIG.2

```
                    START

              MEASURE NEW SPECIMEN         ⟿ S1

            GENERATE B-MODE IMAGE DATA     ⟿ S2

              DISPLAY B-MODE IMAGE         ⟿ S3

               FREQUENCY ANALYSIS          ⟿ S4

              EXTRACT PRE-CORRECTION
                  FEATURE DATA             ⟿ S5

               EXECUTE ATTENUATION
                CORRECTION TO PRE-         ⟿ S6
              CORRECTION FEATURE DATA

              CALCULATE DEGREE OF
                  ASSOCIATION              ⟿ S7

           GENERATE CALCULATION RESULT
                DISPLAY IMAGE DATA         ⟿ S8

            DISPLAY CALCULATION RESULT
                  DISPLAY IMAGE            ⟿ S9

                     END
```

# FIG.3

# FIG.4

FREQUENCY
ANALYSIS

$L = L_0$ — S21

$Z = Z_0$ — S22

ACQUIRE FFT DATA GROUP — S23

WORK WINDOW FUNCTION — S24

S25 — IS
FFT DATA GROUP
NORMAL? — NO

YES

S26 — INSERT ZERO DATA
BY DEFICIENCY

FFT CALCULATION — S27

$Z = Z+D$ — S28

S29 — $Z > Z_{max}$? — NO

YES

$L = L+1$ — S30

NO — S31 — $L > L_{max}$?

YES

RETURN

# FIG.5

# FIG.6

# FIG.7

# FIG.8

# FIG.9

```
        ┌──────────────────────────────┐
        │  CALCULATION OF DEGREE        │
        │      OF ASSOCIATION           │
        └──────────────┬───────────────┘
                       │
        ┌──────────────▼───────────────┐
        │  SET FEATURE DATA SPACE       │───S41
        └──────────────┬───────────────┘
                       │
        ┌──────────────▼───────────────┐
        │  CALCULATE DISTANCE ON FEATURE│
        │  DATA SPACE BETWEEN SPECIMEN  │
        │  POINT AND KNOWN SPECIMEN     │───S42
        │  AVERAGE POINT OF EACH GROUP  │
        └──────────────┬───────────────┘
                       │
        ┌──────────────▼───────────────┐
        │  OUTPUT CALCULATION RESULT    │───S43
        └──────────────┬───────────────┘
                       │
        ┌──────────────▼───────────────┐
        │           RETURN              │
        └──────────────────────────────┘
```

# FIG.10

# FIG.11

# FIG.12

# FIG.13

300

300r

300g

# FIG.14

300

300r

300g

# FIG.15

# FIG.16

```
        ┌─────────────┐
        │    START    │
        └─────────────┘
               │
               ▼
   ┌───────────────────────────┐
   │   MEASURE NEW SPECIMEN     │─── S51
   └───────────────────────────┘
               │
               ▼
   ┌───────────────────────────┐
   │  GENERATE B-MODE IMAGE DATA │─── S52
   └───────────────────────────┘
               │
               ▼
   ┌───────────────────────────┐
   │    DISPLAY B-MODE IMAGE    │─── S53
   └───────────────────────────┘
               │
               ▼
   ┌───────────────────────────┐
   │     FREQUENCY ANALYSIS     │─── S54
   └───────────────────────────┘
               │
               ▼
   ┌───────────────────────────┐
   │     EXECUTE ATTENUATION    │
   │  CORRECTION TO FREQUENCY   │─── S55
   │         SPECTRUM           │
   └───────────────────────────┘
               │
               ▼
   ┌───────────────────────────┐
   │    EXTRACT FEATURE DATA    │─── S56
   └───────────────────────────┘
               │
               ▼
   ┌───────────────────────────┐
   │    CALCULATE DEGREE OF     │─── S57
   │        ASSOCIATION         │
   └───────────────────────────┘
               │
               ▼
   ┌───────────────────────────┐
   │ GENERATE CALCULATION RESULT │
   │     DISPLAY IMAGE DATA     │─── S58
   └───────────────────────────┘
               │
               ▼
   ┌───────────────────────────┐
   │  DISPLAY CALCULATION RESULT │
   │       DISPLAY IMAGE        │─── S59
   └───────────────────────────┘
               │
               ▼
        ┌─────────────┐
        │     END     │
        └─────────────┘
```

# FIG.17

# FIG.18

# FIG.19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060079780 A1

- WO 2005122906 A